# EUROPEAN PATENT APPLICATION

(11) **EP 4 268 847 A1**
(43) Date of publication of application: **01.11.2023**
(21) Application number: 21910847.9
(22) Date of filing: 22.12.2021
(51) Int. Cl.: A61K 39/395, A61P 25/00, C07K 16/40, C12N 15/13

(54) **PHARMACEUTICAL COMPOSITION FOR TREATMENT OF AMYOTROPHIC LATERAL SCLEROSIS**

(30) Priority: 24.12.2020 JP 2020214739
(71) Applicant: Eisai R&D Management Co., Ltd, Bunkyo-ku, Tokyo 112-8088 (JP)
(72) Inventor: TASHIRO, Katsuhisa, Kobe-shi, Hyogo 650-0047 (JP); TAGUCHI, Ryota, Tsukuba-shi, Ibaraki 300-2635 (JP)
(74) Representative: Hoffmann Eitle
(86) International application number: PCT/JP2021/047532
(87) International publication number: WO 2022/138707

(57) **Abstract**

A novel pharmaceutical composition for treating ALS is provided comprising as the active ingredient an anti-EphA4 antibody that can bind to EphA4 and enhance the cleavage of EphA4. A pharmaceutical composition for treating ALS comprising an anti-EphA4 antibody is provided, wherein the anti-EphA4 antibody comprises a heavy chain comprising a heavy chain CDR1 consisting of the amino acid sequence shown in SEQ ID NO. 44, a heavy chain CDR2 consisting of the amino acid sequence shown in SEQ ID NO. 27, and a heavy chain CDR3 consisting of the amino acid sequence shown in SEQ ID NO. 28, and a light chain comprising a light chain CDR1 consisting of the amino acid sequence shown in SEQ ID NO. 29, a light chain CDR2 consisting of the amino acid sequence shown in SEQ ID NO. 30, and a light chain CDR3 consisting of the amino acid sequence shown in SEQ ID NO. 31.

## Description

### Technical Field

The present disclosure relates to a pharmaceutical composition for treating amyotrophic lateral sclerosis (ALS) comprising an antibody that binds to EphA4.

### Background Art

EphA4 is a member of the receptor-type tyrosine kinase family. Ephrin type A and type B are known as the ligands of EphA4, and a de-adhesion signal is induced when EphA4 binds to its ligand ephrin. EphA4 is expressed in motor neurons, and accurate axon guidance is controlled by expression of ephrin at the non-projection region of motor neurons in the spinal cord during the neural circuit forming phase. EphA4 is known to be cleaved neural activity-dependently by matrix metalloprotease (MMP), ADAM (a disintegrin and metalloproteinase), and γ-secretase.

From conventional research, functional inhibition of EphA4 has been suggested to be an effective therapy means for neurodegenerative diseases such as amyotrophic lateral sclerosis (Amyotrophic lateral sclerosis, hereinafter also referred to as "ALS") or Alzheimer's disease and spinal cord injury.

EphA4 has been reported to be the gene that adjusts the phonotype of ALS (Patent Literature 1, Non-Patent Literature 1). It has been shown that genetic deficiency of EphA4 or antagonism by e.g. EphA4-Fc promotes axon growth or functional restoration upon spinal cord injury in mice and rats (Non-Patent Literatures 2 and 3).

KYL peptide or Compound 1 are known as preexisting EphA4 inhibitors (Patent Literature 1, Non-Patent Literatures 1 and 2) . Although antibodies having activity to inhibit the binding between EphA4 and its ligand are also known (Patent Literatures 2 and 3), an antibody having activity to enhance the cleavage of EphA4 has thus far not been reported.

### Citation List

### Patent Literatures

[Patent Literature 1] WO2012/156351 A1
[Patent Literature 2] WO2016/019280 A1
[Patent Literature 3] WO2017/043466 A1

### Non-Patent Literatures

[Non-Patent Literature 1] Van Hoecke et al. , Nature Medicine, vol18: 1418-1422, 2012
[Non-Patent Literature 2] Goldshmit et al., PLoS one, vol6: e24636, 2011
[Non-Patent Literature 3] Spanevello et al., Journal of Neurotrauma,vol30: 1023-1034, 2013

### Summary of the Invention

### Problems to be Solved by the Invention

The object of the present disclosure is to provide a novel pharmaceutical composition for treating ALS.

### Means for Solving the Problems

As a result of extensive investigation to solve the above problem, the present inventors have come to acquire an antibody effective for ALS therapy that can bind to EphA4 and enhance the cleavage of EphA4.

The present disclosure encompasses the following characteristics.
(1) A pharmaceutical composition for treating amyotrophic lateral sclerosis (ALS) comprising an anti-EphA4 antibody, wherein the anti-EphA4 antibody comprises a heavy chain comprising:
   (a) a heavy chain CDR1 consisting of the amino acid sequence shown in SEQ ID NO. 44;
   (b) a heavy chain CDR2 consisting of the amino acid sequence shown in SEQ ID NO. 27; and
   (c) a heavy chain CDR3 consisting of the amino acid sequence shown in SEQ ID NO. 28; and
   a light chain comprising:
   (d) a light chain CDR1 consisting of the amino acid sequence shown in SEQ ID NO. 29;
   (e) a light chain CDR2 consisting of the amino acid sequence shown in SEQ ID NO. 30; and
   (f) a light chain CDR3 consisting of the amino acid sequence shown in SEQ ID NO. 31.
(2) The pharmaceutical composition according to (1), wherein the anti-EphA4 antibody is humanized.
(3) The pharmaceutical composition according to (1) or (2), wherein the anti-EphA4 antibody specifically binds to EphA4 and enhances the cleavage of EphA4.
(4) The pharmaceutical composition according to any of (1) to (3), wherein the anti-EphA4 antibody specifically binds to EphA4 and inhibits the binding between EphA4 and ephrin.
(5) The pharmaceutical composition according to any of (1) to (4), wherein
   the heavy chain comprises a variable region consisting of the amino acid sequence shown in SEQ ID NO. 45, and
   the light chain comprises a variable region consisting of the amino acid sequence shown in SEQ ID NO. 46.
(6) The pharmaceutical composition according to any of (1) to (5), wherein the constant region of the heavy chain and the constant region of the light chain comprise an amino acid sequence derived from a human antibody.
(7) The pharmaceutical composition according to (6), wherein the constant region of the heavy chain is the constant region of human IgG.
(8) The pharmaceutical composition according to (7), wherein the constant region of human IgG is the constant region of human IgG₂.
(9) The pharmaceutical composition according to (8), wherein the constant region of human IgG₂ comprises the amino acid sequence shown in SEQ ID NO. 47.
(10) The pharmaceutical composition according to any of (6) to (9), wherein the constant region of the light chain is the constant region of human Igκ.
(11) The pharmaceutical composition according to (10), wherein the constant region of human Igκ comprises the amino acid sequence shown in SEQ ID NO. 48.
(12) A pharmaceutical composition for treating amyotrophic lateral sclerosis (ALS) comprising an anti-EphA4 antibody, wherein
   the anti-EphA4 antibody comprises a heavy chain and a light chain,
   the heavy chain comprises the amino acid sequence shown in SEQ ID NO. 59,
   the light chain comprises the amino acid sequence shown in SEQ ID NO. 60, and
   the C-terminal lysine of the heavy chain may be optionally deleted.
(13) The pharmaceutical composition according to (12), wherein the C-terminal lysine of the heavy chain is deleted.
(14) A pharmaceutical composition for treating amyotrophic lateral sclerosis (ALS) comprising an anti-EphA4 antibody, wherein
   the anti-EphA4 antibody comprises a heavy chain and a light chain,
   the heavy chain comprises the amino acid sequence shown in SEQ ID NO. 59, and
   the light chain comprises the amino acid sequence shown in SEQ ID NO. 60.
(15) A pharmaceutical composition for treating amyotrophic lateral sclerosis (ALS) comprising an anti-EphA4 antibody, wherein
   the anti-EphA4 antibody comprises a heavy chain and a light chain,
   the heavy chain comprises the amino acid sequence shown in SEQ ID NO. 59,
   the light chain comprises the amino acid sequence shown in SEQ ID NO. 60, and
   the C-terminal lysine of the heavy chain is deleted.
(16) The pharmaceutical composition according to any of (1) to (15), further comprising at least one pharmaceutically acceptable carrier.
(17) An anti-EphA4 antibody, wherein
   the anti-EphA4 antibody comprises a heavy chain comprising:
      (a) a heavy chain CDR1 consisting of the amino acid sequence shown in SEQ ID NO. 44;
      (b) a heavy chain CDR2 consisting of the amino acid sequence shown in SEQ ID NO. 27; and
      (c) a heavy chain CDR3 consisting of the amino acid sequence shown in SEQ ID NO. 28; and
   a light chain comprising:
      (d) a light chain CDR1 consisting of the amino acid sequence shown in SEQ ID NO. 29;
      (e) a light chain CDR2 consisting of the amino acid sequence shown in SEQ ID NO. 30; and
      (f) a light chain CDR3 consisting of the amino acid sequence shown in SEQ ID NO. 31.
(18) The anti-EphA4 antibody according to (17), wherein the anti-EphA4 antibody is humanized.
(19) The anti-EphA4 antibody according to (17) or (18), wherein the anti-EphA4 antibody specifically binds to EphA4 and enhances the cleavage of EphA4.
(20) The anti-EphA4 antibody according to any of (17) to (19), wherein the anti-EphA4 antibody specifically binds to EphA4 and inhibits the binding between EphA4 and ephrin.
(21) The anti-EphA4 antibody according to any of (17) to (20), wherein
   the heavy chain comprises a variable region consisting of the amino acid sequence shown in SEQ ID NO. 45, and
   the light chain comprises a variable region consisting of the amino acid sequence shown in SEQ ID NO. 46.
(22) The anti-EphA4 antibody according to any of (17) to (21), wherein the constant region of the heavy chain and the constant region of the light chain comprise an amino acid sequence derived from a human antibody.
(23) The anti-EphA4 antibody according to (22), wherein the constant region of the heavy chain is the constant region of human IgG.
(24) The anti-EphA4 antibody according to (23), wherein the constant region of human IgG is the constant region of human IgG₂.
(25) The anti-EphA4 antibody according to (24), wherein the constant region of human IgG₂ comprises the amino acid sequence shown in SEQ ID NO. 47.
(26) The anti-EphA4 antibody according to any of (22) to (25), wherein the constant region of the light chain is the constant region of human Igκ.
(27) The anti-EphA4 antibody according to (26), wherein the constant region of human Igκ comprises the amino acid sequence shown in SEQ ID NO. 48.
(28) An anti-EphA4 antibody, wherein
   the anti-EphA4 antibody comprises a heavy chain and a light chain,
   the heavy chain comprises the amino acid sequence shown in SEQ ID NO. 59,
   the light chain comprises the amino acid sequence shown in SEQ ID NO. 60, and
   the C-terminal lysine of the heavy chain may be optionally deleted.
(29) The anti-EphA4 antibody according to (28), wherein the C-terminal lysine of the heavy chain is deleted.
(30) An anti-EphA4 antibody, wherein
   the anti-EphA4 antibody comprises a heavy chain and a light chain,
   the heavy chain comprises the amino acid sequence shown in SEQ ID NO. 59, and
   the light chain comprises the amino acid sequence shown in SEQ ID NO. 60.
(31) An anti-EphA4 antibody, wherein
   the anti-EphA4 antibody comprises a heavy chain and a light chain,
   the heavy chain comprises the amino acid sequence shown in SEQ ID NO. 59,
   the light chain comprises the amino acid sequence shown in SEQ ID NO. 60, and
   the C-terminal lysine of the heavy chain is deleted.
(32) The anti-EphA4 antibody according to any of (17) to (31) for use in treating amyotrophic lateral sclerosis (ALS).
(33) A method for treating amyotrophic lateral sclerosis (ALS), comprising administering to a patient in need thereof a therapeutically effective amount of the anti-EphA4 antibody according to any of (17) to (31).
(34) The use of the anti-EphA4 antibody according to any of (17) to (31) for manufacturing a pharmaceutical composition for treating amyotrophic lateral sclerosis (ALS).
(35) The use of the anti-EphA4 antibody according to (34), wherein the pharmaceutical composition comprises at least one pharmaceutically acceptable carrier.
(36) A therapeutic agent for amyotrophic lateral sclerosis (ALS) comprising the anti-EphA4 antibody according to any of (17) to (31).

### Effects of the Invention

According to the present disclosure, a novel pharmaceutical composition for treating ALS is provided. The pharmaceutical composition comprises as the active ingredient an anti-EphA4 antibody that can bind to EphA4 and enhance the cleavage of EphA4.

### Brief Description of the Drawings

Figure 1 shows the binding affinity of anti-EphA4 monoclonal antibody (antibody A) against mouse and human EphA4.
Figure 2 shows the EphA4 cleavage enhancing activity of anti-EphA4 monoclonal antibody (antibody A) employing hippocampus neurons.
Figure 3 shows the mouse EphA4-mouse ligand binding inhibitory activity of anti-EphA4 monoclonal antibody (antibody A).
Figure 4 shows the human EphA4-human ligand binding inhibitory activity of anti-EphA4 monoclonal antibody (antibody A).
Figure 5 shows the selectivity of anti-EphA4 monoclonal antibody (antibody A) against each human Eph receptor.
Figure 6 shows the selectivity of anti-EphA4 monoclonal antibody (antibody A) against each mouse Eph receptor.
Figure 7 shows the reactivity of anti-EphA4 monoclonal antibody (antibody A) against mouse, rat, monkey, and human EphA4.
Figure 8 shows the reactivity of anti-EphA4 monoclonal antibody (antibody A) against human EphA4 extracellular region (ECD), ligand binding domain (LBD), fibronectin type III domain 1 (FN1), and fibronectin type III domain 2 (FN2).
Figure 9 shows the increasing effect against the number of spines in the hippocampus neuron by anti-EphA4 monoclonal antibody (antibody A).
Figure 10A shows the amino acids of the EphA4-Ligand Binding Domain (EphA4-LBD) on the horizontal axis, and the structural region of antibody A-Fab on the vertical axis. The black bits show the intersection of the combination where interaction exists.
Figure 10B shows the surface structure of the EphA4-Ligand Binding Domain (EphA4-LBD). In Figure 10B, name and residue number of the amino acids contained in the binding region are shown at the corresponding positions, and the CDRs of the H chain and L chain of the binding antibody A-Fab are shown in a ribbon model.
Figure 11 shows the affinity of humanized anti-EphA4 monoclonal antibody (antibody B) against human EphA4.
Figure 12 shows the EphA4 cleavage enhancing activity of humanized anti-EphA4 monoclonal antibody (antibody B) in hippocampus neurons.
Figure 13 shows the human EphA4-human ligand binding inhibitory activity of humanized anti-EphA4 monoclonal antibody (antibody B).
Figure 14 shows the mouse EphA4-mouse ligand binding inhibitory activity of humanized anti-EphA4 monoclonal antibody (antibody B).
Figure 15 shows the selectivity of humanized anti-EphA4 monoclonal antibody (antibody B) against human Eph receptor.
Figure 16 shows the selectivity of humanized anti-EphA4 monoclonal antibody (antibody B) against mouse Eph receptor.
Figure 17 shows the reactivity of humanized anti-EphA4 monoclonal antibody (antibody B) against mouse, rat, monkey, and human EphA4.
Figure 18 shows the reactivity of humanized anti-EphA4 monoclonal antibody (antibody B) against human EphA4 extracellular region (ECD), ligand binding domain (LBD), fibronectin type III domain 1 (FN1), and fibronectin type III domain 2 (FN2).
Figure 19 shows the increasing effect against the number of spines in the hippocampus neuron by humanized anti-EphA4 monoclonal antibody (antibody B).
Figure 20 shows the human EphA4 cleavage enhancing activity of humanized anti-EphA4 monoclonal antibody (antibody B) in hippocampus neurons.
Figure 21 shows the increasing effect against the number of spines in the hippocampus neuron via MMP and ADAM by humanized anti-EphA4 monoclonal antibody (antibody B).
Figure 22 shows the schematic diagram of the evaluation system carried out in Example 13.
Figure 23 shows the effect of humanized anti-EphA4 monoclonal antibody (antibody B) on human iPS cell-derived motor neuron death induced by mutated human SOD1 (G93A)-expressing astrocytes.

### Description of Embodiments

The regions specified or coded by the SEQ ID NOs. used herein are as follows:

| SEQ No | Sequence Region | SEQ No | Sequence Region |
|---|---|---|---|
| 1 | Mouse EphA4 (amino acid sequence) | 34 | Signal sequence of human EphA4 (amino acid sequence) |
| 2 | Extracellular region of mouse EphA4 (amino acid sequence) | 35 | Signal sequence of preprotrypsin (amino acid sequence) |
| 3 | Mouse EphA4 extracellular region-SEAP-His protein (amino acid sequence) | 36 | Extracellular region of human EphA4 (amino acid sequence) |
| 4 | Signal sequence of mouse EphA4 (amino acid sequence) | 37 | Ligand binding domain of human EphA4 (amino acid sequence) |
| 5 | Human EphA4 (amino acid sequence) | 38 | Fibronectin type III domain 1 of human EphA4 (amino acid sequence) |
| 6 | Extracellular region of human EphA4 (amino acid sequence) | 39 | Fibronectin type III domain 2 of human EphA4 (amino acid sequence) |
| 7 | Oligo DNA ad29S (nucleic acid sequence) | 40 | Light chain FR IGKV1-17*01 of human antibody (amino acid sequence) |
| 8 | Oligo DNA ad29AS (nucleic acid sequence) | 41 | Light chain FR JK4 of human antibody (amino acid sequence) |
| 9 | 5' forward primer (nucleic acid sequence) | 42 | Heavy chain FR IGHV3-33*03 of human antibody (amino acid sequence) |
| 10 | 3' reverse primer for mouse IgG heavy chain (nucleic acid sequence) | 43 | Heavy chain FR JH6 of human antibody (amino acid sequence) |
| 11 | 3' reverse primer for mouse Igκ light chain (nucleic acid sequence) | 44 | Heavy chain CDR1 of antibody B (amino acid sequence) |
| 12 | Heavy chain signal sequence of antibody A (amino acid sequence) | 45 | Heavy chain variable region HK2-42 of antibody B (amino acid sequence) |
| 13 | Heavy chain variable region of antibody A (amino acid sequence) | 46 | Light chain variable region L1-8 of antibody B (amino acid sequence) |
| 14 | Light chain signal sequence of antibody A (amino acid sequence) | 47 | Human IgG₂ heavy chain constant region of antibody B (amino acid sequence) |
| 15 | Light chain variable region of antibody A (amino acid sequence) | 48 | Human Igκ light chain constant region of antibody B (amino acid sequence) |
| 16 | Heavy chain signal sequence of antibody A (nucleic acid sequence) | 49 | Heavy chain CDR1 of antibody B (nucleic acid sequence) |
| 17 | Heavy chain variable region of antibody A (nucleic acid sequence) | 50 | Heavy chain CDR2 of antibody B (nucleic acid sequence) |
| 18 | Light chain signal sequence of antibody A (nucleic acid sequence) | 51 | Heavy chain CDR3 of antibody B (nucleic acid sequence) |
| 19 | Light chain variable region of antibody A (nucleic acid sequence) | 52 | Light chain CDR1 of antibody B (nucleic acid sequence) |
| 20 | 5' forward primer for antibody A heavy chain (nucleic acid sequence) | 53 | Light chain CDR2 of antibody B (nucleic acid sequence) |
| 21 | 5' forward primer for antibody A light chain (nucleic acid sequence) | 54 | Light chain CDR3 of antibody B (nucleic acid sequence) |
| 22 | 3' reverse primer for antibody A heavy chain (nucleic acid sequence) | 55 | Heavy chain variable region HK2-42 of antibody B (nucleic acid sequence) |
| 23 | 3' reverse primer for antibody A light chain (nucleic acid sequence) | 56 | Light chain variable region L1-8 of antibody B (nucleic acid sequence) |
| 24 | Heavy chain constant region of antibody A (amino acid sequence) | 57 | Human IgG₂ heavy chain constant region of antibody B (nucleic acid sequence) |
| 25 | Light chain constant region of antibody A (amino acid sequence) | 58 | Human Igκ light chain constant region of antibody B (nucleic acid sequence) |
| 26 | Heavy chain CDR1 of antibody A (amino acid sequence) | 59 | Heavy chain full length sequence of antibody B (amino acid sequence) |
| 27 | Heavy chain CDR2 of antibody A (amino acid sequence) | 60 | Light chain full length sequence of antibody B (amino acid sequence) |
| 28 | Heavy chain CDR3 of antibody A (amino acid sequence) | 61 | Heavy chain full length sequence of antibody B (nucleic acid sequence) |
| 29 | Light chain CDR1 of antibody A (amino acid sequence) | 62 | Light chain full length sequence of antibody B (nucleic acid sequence) |
| 30 | Light chain CDR2 of antibody A (amino acid sequence) | 63 | Primer for mutated human SOD1 (nucleic acid sequence) |
| 31 | Light chain CDR3 of antibody A (amino acid sequence) | 64 | Primer for mutated human SOD1 (nucleic acid sequence) |
| 32 | Monkey EphA4 (amino acid sequence) | 65 | Primer for internal standard (nucleic acid sequence) |
| 33 | Extracellular region of monkey EphA4 (amino acid sequence) | 66 | Primer for internal standard (nucleic acid sequence) |

The anti-EphA4 antibody according to the present disclosure is an antibody that can recognize and bind to EphA4, and as described below, the aforementioned antibody may be an intact antibody, or may be a synthetic antibody (such as a recombinant antibody, a chimeric antibody, and a humanized antibody) as long as it has binding affinity with EphA4. In the present specification, EphA4 can be recognized as referring to a human, mouse, rat, and monkey-derived EphA4. The human, mouse, rat, and monkey-derived EphA4 can be obtained from a public database where sequence information is registered, such as Genbank provided by U.S. National Center for Biotechnology Information. In addition, the sequence information of EphA4 gene can be obtained by designing a primer based on the base sequence information of EphA4 of a closely related animal species, and cloning from RNA extracted from the desired animal species. For example, the base sequence information of human, mouse, rat, and monkey EphA4 are registered in the database as Genbank Accession No. NM_004438.5, NM_007936.3, NM_001162411.1, NM_001260870.1, respectively.

In one aspect, the anti-EphA4 antibody is an antibody that specifically binds to EphA4. The term "specific binding" is a term well-known to those skilled in the art in the aforementioned technical field, and methods for determining the specific binding of an antibody or an antigen-binding fragment thereof towards an antigen or an epitope are also well-known. In one embodiment, "specific binding" is recognized as that the anti-EphA4 antibody can bind to EphA4 by immunological reaction at a larger binding affinity and binding activity, more rapidly, and/or for a longer duration of time compared to binding with other target molecules. This does not mean that an antibody that specifically binds to EphA4 does not bind to other target molecules. In another embodiment, "specific binding" may be indicated by an antibody that has a KD of at least about 10⁻⁷ M, or at least about 10⁻⁸ M, or at least about 10⁻⁹ M, or lower, against EphA4. Moreover, in yet another embodiment, "specific binding" is recognized as binding to EphA4 by immunological reaction but substantially do not bind to other family molecules of the Eph receptor.

In one aspect, the anti-EphA4 antibody is an antibody that binds to the extracellular region of EphA4. In one embodiment, the anti-EphA4 antibody is an antibody that binds to the ligand binding domain (LBD) among the extracellular regions of EphA4.

In one embodiment, the anti-EphA4 antibody can specifically bind to EphA4 and enhance the cleavage of EphA4. In a particular embodiment, the anti-EphA4 antibody can specifically bind to EphA4 and enhance the cleavage of EphA4 extracellular domain by matrix metalloprotease (MMP) or ADAM (a disintegrin and metalloproteinase).

In one embodiment, the anti-EphA4 antibody can specifically bind to EphA4 and inhibit the binding between EphA4 and its ligand ephrin.

In another embodiment, the anti-EphA4 antibody can specifically bind to EphA4 and increase the number of spines in the hippocampus neuron or stabilize the spines of the hippocampus neuron.

In another embodiment, the anti-EphA4 antibody can protect motor neurons from cell death caused by SOD1 gene abnormality.

In one embodiment, the present disclosure encompasses an anti-EphA4 antibody that can specifically bind to at least one of human EphA4, mouse EphA4, rat EphA4, and monkey EphA4 and inhibit the binding with its ligand. In another embodiment, the present disclosure encompasses an anti-EphA4 antibody that can specifically bind to two or more of human EphA4, mouse EphA4, rat EphA4, and monkey EphA4 and inhibit the binding with its ligand. In yet another embodiment, the present disclosure encompasses an anti-EphA4 antibody that can specifically bind to all of human EphA4, mouse EphA4, rat EphA4, and monkey EphA4 and inhibit the binding with its ligand.

For the method for measuring the binding property of anti-EphA4 antibody towards the antigen (such as binding affinity and cross-species reactivity), a method well-known to those skilled in the art in the aforementioned technical field may be employed. For example, binding affinity may be measured using, but not limited to, Biacore^{™} biosensor, KinExA biosensor, scintillation proximity assay, ELISA, ORIGEN immunoassay (from IGEN), flow cytometry, fluorescence quenching, fluorescence metastasis, yeast display, and/or immunostaining. The neutralizing activity of anti-EphA4 antibody against the binding between EphA4 and its ligand may be measured using, but not limited to, Biacore^{™} biosensor, ELISA, and/or flow cytometry.

The anti-EphA4 antibody according to the present disclosure may be a monoclonal antibody as long as it binds to EphA4.

The anti-EphA4 antibody according to the present disclosure may be of any class such as IgG, IgA, or IgM (or a subclass thereof), and is not limited to a particular class. Immunoglobulins are classified into different classes by the antibody amino acid sequence of the constant region of the heavy chain (sometimes also referred to as the H chain). There are five major immunoglobulin classes: IgA, IgD, IgE, IgG, and IgM, some of which may be further subdivided into subclasses (isotypes) such as IgG₁ IgG₂, IgG₃, IgG₄, IgAi, and IgA₂. The corresponding constant region of the heavy chain of the different classes of immunoglobulin are each called α, δ, ε, γ, and µ. Moreover, the types of the light chain (sometimes also referred to as the L chain) of an antibody are λ chain and κ chain. The anti-EphA4 antibody according to the present disclosure may be an IgG antibody, for example may be an IgG₁ antibody or an IgG₂ antibody and the like. Moreover, the anti-EphA4 antibody according to the present disclosure may be in some cases in the form of a monomer, a dimer, or a multimer.

The variable region of the antibody according to the present disclosure may mean the variable region of the antibody light chain and/or the variable region of the antibody heavy chain, and the constant region of the antibody may mean the constant region of the antibody light chain and/or the constant region of the antibody heavy chain. The variable regions of the heavy and light chains each consist of four framework regions (FR) linked by three CDRs also known as complementarity-determining regions. The CDRs in each chain are retained in proximity by FRs, and together with the CDRs in the other chain, contribute to the formation of the antigen binding site of the antibody. Technologies for determining CDRs include, but are not limited to, e.g. (1) an approach based on cross-species sequence variability (such as Kabat et al, Sequences of Proteins of Immunological Interest, 5th ed., 1991, National Institutes of Health, Bethesda MD); and (2) an approach based on crystal structure research of antigen-antibody complex (Al-lazikani et al., 1997 J. Molec. Biol. 273:927-948). These and other approaches may be employed in combination.

In the present specification, a monoclonal antibody may mean an antibody that is obtained from a substantially uniform antibody population. In other words, the individual antibodies contained in the population are identical except for a few possible natural mutants that may exist. A monoclonal antibody is directed towards a single antigenic site and is extremely specific. Further, in contrast to a typical polyclonal antibody that targets different antigens or different epitopes, each monoclonal antibody targets a single epitope of an antigen. The modifier "monoclonal" indicates the property of an antibody that is obtained from a substantially uniform antibody population, and is not to be construed as limiting as requiring the production of the antibody by a particular method.

The anti-EphA4 antibody according to the present disclosure may be a mouse antibody, a chimeric antibody, or a humanized antibody. A chimeric antibody is for example an antibody having the variable region of a non-human (such as mouse or rat) antibody fused with the constant region of a human antibody, and for example may refer to an antibody where the variable region is derived from a non-human antibody and the constant region is derived from a human antibody. A humanized antibody is for example an antibody having the complementarity-determining region (CDR (sometimes referred to as the hypervariable region)) of a non-human antibody introduced into a human antibody, and for example may refer to an antibody where the CDR is derived from a non-human antibody and the remaining antibody regions are derived from a human antibody. Note that the boundary between a chimeric antibody and a humanized antibody does not necessarily need to be definite, and it may be in a state that may be called a chimeric antibody or a humanized antibody. Moreover, in a chimeric antibody or a humanized antibody, the human antibody-derived antibody regions (FR, constant region) does not necessarily need to be all composed of amino acids derived from the human antibody, and may comprise one or more non-human antibody-derived amino acids as long as it can be used normally in a human subject. One embodiment of a humanized antibody is an antibody where the CDR is derived from a rodent antibody and the remaining antibody regions are derived from a human antibody. A particular embodiment of a humanized antibody is an antibody where the CDR is derived from a mouse antibody and the remaining antibody regions are derived from a human antibody. In these embodiments, the CDR may comprise amino acids derived from one or more non-rodent antibodies or amino acids derived from one or more non-mouse antibodies, and the antibody regions other than the CDR may comprise amino acids derived from one or more non-human antibodies. Here, "more" is, but is not limited to, 2 to 20, or 2 to 15, such as 14, 13, 12, 11, 10, 9, 8, 7, 6, 5, 4, 3, or 2, or within 10%, within 9%, within 8%, within 7%, within 6%, within 5%, within 4%, within 3%, within 2%, or within 1% of the number of amino acids in the amino acid sequence. Humanization can be carried out with a CDR transplantation method (Kontermann and Dubel, Antibody Engineering, Springer Lab Manual (2001) and Tsurushita et al., Methods 36:69-83 (2005)), or it can also be carried out by substituting the CDR sequence with a corresponding sequence of the human antibody with a method well-known in the aforementioned technical field (see e.g. Jones et al., Nature 321:522-525 (1986); Riechmann et al., Nature 332:323-327 (1988); and Verhoeyen et al., Science 239:1534-1536 (1988)).

In order to decrease antigenicity, it may be important to select the use of human variable region for both light and heavy chains in generating a humanized antibody. According to the "best-fit" method, the sequence of the variable region of a rodent antibody is screened against the entire library of known human FR sequences. Next, the human sequence that is the closest to the rodent sequence is accepted as the human FR of the humanized antibody. See e.g. Sims et al., J. Immunol. 151:2296-2308 (1993) and Chothia et al., J. Mol. Biol. 196:901-917 (1987). In another method, a particular framework derived from the common sequence of the entire human antibody of a particular subgroup of the light chain or the heavy chain is employed. The same framework may be employed for several different humanized antibodies. See e.g. Carter et al., Proc. Natl. Acad. Set USA 89:4285-4289 (1992) and Presta et al., J. Immunol. 151: 2623-2632 (1993).

Further, it is desired that a humanized antibody generally retains high binding affinity against the antigen and other preferred biological natures. To this end, according to one method, a humanized antibody is prepared by a step of analyzing the parent sequence and various conceptual humanized products employing three-dimensional models of the parent and the humanized sequences. Three-dimensional immunoglobulin models are generally available, and are known to those skilled in the art. Computer programs that illustrate and display a promising three-dimensional conformation of the immunoglobulin sequence which is the selected candidate are available. By investigating these displays, analysis of the possible roles of residues in the function of the candidate immunoglobulin sequence, i.e. analysis of residues that influence the ability of the candidate immunoglobulin to bind to its antigen will be possible. By this method, FR residues can be selected from the recipient sequence and the import sequence and combined, so that desirable antibody properties such as the increase in binding affinity against a single or multiple target antigens (such as EphA4 or a fragment thereof) are achieved.

Needless to say, an antibody where the chimeric antibody or the humanized antibody illustrated above was appropriately altered (such as modification of the antibody or partial substitution, addition, and/or deletion of the amino acid sequence of the antibody) while retaining the function of the aforementioned antibody (alternatively, in order to add to or improve the function of the aforementioned antibody) is also included in the anti-EphA4 antibody according to the present disclosure. More specifically, an antibody where the amino acid sequence of the constant region was altered in order to modify the effector function of the antibody is also included in the scope of the present disclosure, and for example, an antibody where valine (Val) at position 234 of human IgG₂ antibody in terms of Eu numbering is substituted to alanine (Ala) and glycine (Gly) at position 237 is substituted to alanine (Ala) in order to reduce antibody-dependent cellular cytotoxicity (ADCC) activity and/or antibody-dependent cell phagocytosis (ADCP) activity and the like is also included in the scope of the present disclosure. Further, a bispecific antibody that has an antibody binding site having the CDR sequence of the anti-EphA4 antibody according to the present disclosure combined together with an antigen binding site that binds to a different antigen (Kontermann (2012), mAbs 4, 182-97) is also included in the scope of the present disclosure.

The anti-EphA4 antibody according to the present disclosure may be modified as desired. The modification of the anti-EphA4 antibody may be a modification that changes (a) the three-dimensional structure of the amino acid sequence in the modification region such as e.g. the sheet or helix conformation; (b) the molecular charge or hydrophobicity state at the target site; or (c) the effect of modification on maintaining the side chain volume, or may be a modification in which these changes are not clearly observed.

The modification of the anti-EphA4 antibody according to the present disclosure may be achieved by e.g. substitution, deletion, addition, and the like of the configuring amino acid residues.

In the present specification, an amino acid is employed in its broadest meaning, and includes not only natural amino acids, e.g. serine (Ser), asparagine (Asn), valine (Val), leucine (Leu), isoleucine (Ile), alanine (Ala), tyrosine (Tyr), glycine (Gly), lysine (Lys), arginine (Arg), histidine (His), aspartic acid (Asp), glutamic acid (Glu), glutamine (Gin), threonine (Thr), cysteine (Cys), methionine (Met), phenylalanine (Phe), tryptophan (Trp), and proline (Pro), but also non-natural amino acids such as amino acid variants and derivatives. Those skilled in the art will naturally recognize that in consideration of this broad definition, the amino acids herein include e.g. L-amino acids; D-amino acids; chemically modified amino acids such as amino acid variants and amino acid derivatives; amino acids that will not be a component of proteins in the body such as norleucine, β-alanine, and ornithine; as well as chemically synthesized compounds having amino acid properties well-known to those skilled in the art, and the like. Examples of non-natural amino acids include α-methylamino acids (such as α-methylalanine), D-amino acids (such as D-aspartic acid and D-glutamic acid), histidine-like amino acids (such as 2-amino-histidine, β-hydroxy-histidine, homohistidine, α-fluoromethyl-histidine, and α-methyl-histidine), amino acids having excess methylenes on the side chain ("homo" amino acids), and amino acids where the carboxylate functional group amino acid in the side chain is substituted with a sulfonate group (such as cysteic acid), and the like.

For example, naturally-occurring amino acid residues may be classified into the following groups based on general side chain properties:
(1) hydrophobic: Met, Ala, Val, Leu, Ile;
(2) neutral hydrophilic: Asn, Gln, Cys, Ser, Thr;
(3) acidic: Asp, Glu;
(4) basic: His, Lys, Arg;
(5) residues that influence chain orientation: Gly, Pro; and
(6) aromatic: Trp, Tyr, Phe.

A nonconservative substitution of an amino acid sequence that configures an antibody may be carried out by exchanging an amino acid belonging to one of these groups with an amino acid belonging to another group. A more conservative substitution may be carried out by exchanging an amino acid belonging to one of these groups with another amino acid in the same group. Similarly, deletion or substitution of an amino acid sequence may be appropriately carried out.

Modification of an amino acid that configures the antibody may be e.g. a post-translational modification such as glycosylation by a sugar, acetylation, or phosphorylation. An antibody may be glycosylated at a conserved position in its constant region. Glycosylation of an antibody is ordinarily either N-linked or O-linked. N-linked means the binding of a sugar portion to the asparagine residue side chain. Tripeptide sequences asparagine-X-serine, asparagine-X-threonine, and asparagine-X-cysteine (wherein X is any amino acid other than proline) are recognition sequences for enzymatically adding a sugar portion to the asparagine side chain. A potential glycosylation site exists by the presence of any of these tripeptide sequences in the antibody. O-linked glycosylation may be the binding of any of N-acetylgalactosamine, galactose, or xylose to a hydroxy amino acid (such as serine or threonine), and in some cases may be the binding to 5-hydroxy proline or 5-hydroxy lysine. Glycosylation conditions (e.g. the type of host cell or cell medium, pH, and the like when glycosylation is carried out by biological means) can be appropriately selected by those skilled in the art according to the purpose.

The anti-EphA4 antibody according to the present disclosure can be further modified based on technical common sense well-known to those skilled in the art by other modification methods alone or in combination.

The anti-EphA4 antibody according to the present disclosure can be produced by methods well-known to those skilled in the art. For example, the antibody may be produced by integrating the nucleic acid encoding the anti-EphA4 antibody according to the present disclosure into an expression vector, introducing the aforementioned expression vector into a host cell, and culturing the aforementioned host cell. Accordingly, the present disclosure encompasses a nucleic acid encoding an anti-EphA4 antibody, a vector comprising the aforementioned nucleic acid, a host cell comprising the aforementioned vector, and a method of generating an anti-EphA4 antibody comprising a step of culturing the aforementioned host cell.

The nucleic acid encoding the anti-EphA4 antibody according to the present disclosure may have a DNA encoding a signal sequence, and may have DNA encoding a signal sequence at the 5'-terminal of the DNA encoding the heavy chain variable region and the DNA encoding the light chain variable region. The signal sequence is an amino acid residue present at the N-terminal of a protein that is necessary for the secretory protein or the integral membrane protein to pass through the lipid bilayer after being synthesized on ribosomes, and in the present disclosure is not particularly limited as long as it is a sequence having this function. Signal sequences that the anti-EphA4 antibody according to the present disclosure may comprise include signal sequences derived from human, mouse, rat, rabbit, donkey, goat, horse, bird, dog, cat, yeast, and the like. In the present disclosure, specifically, signal sequences related to the heavy chain can include peptides comprising amino acid sequences represented by SEQ ID NO. 12 or 16, and signal sequences related to the light chain can include peptides comprising amino acid sequences represented by SEQ ID NO. 14 or 18. Moreover, the amino acid sequence represented by SEQ ID NO. 12 or 16 and an amino acid sequence represented by SEQ ID NO. 14 or 18 may have substitution, addition, and/or deletion of one or more (such as 2, 3, 4, or 5) amino acids if it is functionally equivalent.

The anti-EphA4 antibody according to the present disclosure may be those isolated or purified according to methods well-known to those skilled in the art.

In the present specification, "isolated" or "purified" means that it is artificially isolated or purified from the natural state. If a molecule or a composition is naturally-occurring, it is "isolated" or "purified" when it is changed or removed from the original environment or both. Examples of isolation or purification methods include electrophoretic, molecular biological, immunological, or chromatographic methods, and the like, and specifically include, but are not limited to, ion exchange chromatography, hydrophobic chromatography, reverse phase HPLC chromatography, isoelectric focusing, or alkali extraction method, and the like.

In one embodiment, the anti-EphA4 antibody comprises the following CDRs:
(a) a heavy chain CDR1 consisting of the amino acid sequence shown in SEQ ID NO. 44;
(b) a heavy chain CDR2 consisting of the amino acid sequence shown in SEQ ID NO. 27;
(c) a heavy chain CDR3 consisting of the amino acid sequence shown in SEQ ID NO. 28;
(d) a light chain CDR1 consisting of the amino acid sequence shown in SEQ ID NO. 29;
(e) a light chain CDR2 consisting of the amino acid sequence shown in SEQ ID NO. 30; and
(f) a light chain CDR3 consisting of the amino acid sequence shown in SEQ ID NO. 31.

In one embodiment, the anti-EphA4 antibody is a humanized antibody or a chimeric antibody, and in a particular embodiment a humanized antibody.

In another embodiment, the anti-EphA4 antibody comprises a heavy chain and a light chain, the heavy chain comprises a variable region consisting of the amino acid sequence shown in SEQ ID NO. 45, and the light chain comprises a variable region consisting of the amino acid sequence shown in SEQ ID NO. 46. Note that in the aforementioned embodiment, the variable region of heavy chain and/or the variable region of light chain may comprise an amino acid sequence where one or more amino acids are substituted, added, and/or deleted in the amino acid sequence shown in SEQ ID NO. 45 and/or the amino acid sequence shown in SEQ ID NO. 46. Here, "more" is not limited as long as the binding affinity against EphA4 is retained and the cleavage of EphA4 is enhanced and is 2 to 15, or 2 to 10, such as 9, 8, 7, 6, 5, 4, 3, or 2, or within 10%, such as within 9%, within 8%, within 7%, within 6%, within 5%, within 4%, within 3%, within 2%, or within 1% of the number of amino acids in the amino acid sequence.

In one embodiment, the heavy chain of the anti-EphA4 antibody comprises the constant region of human IgG₂.

In a particular embodiment, the constant region of human IgG₂ comprises the amino acid sequence of SEQ ID NO. 47.

In one embodiment, the light chain of the anti-EphA4 antibody comprises the constant region of human Igκ.

In a particular embodiment, the constant region of human Igκ comprises the amino acid sequence of SEQ ID NO. 48.

In one embodiment, anti-EphA4 antibody comprises a heavy chain comprising the amino acid sequence shown in SEQ ID NO. 59 and a light chain comprising the amino acid sequence shown in SEQ ID NO. 60.

In another embodiment, for example for reasons of decreasing the ununiformity of antibodies produced by antibody-producing cells (U.S. Patent Application Publication No. 2010/0297697 or Liu H et al., MAbs. 2014 Sep-Oct; 6 (5): 1145-1154), anti-EphA4 antibody has the lysine positioned at the C-terminal (carboxy terminal) of the heavy chain deleted. In the present disclosure, the anti-EphA4 antibody where the C-terminal lysine of the heavy chain is deleted includes an anti-EphA4 antibody where the C-terminal lysine of the heavy chain is deleted by genetic modification or an anti-EphA4 antibody where the C-terminal lysine of the heavy chain is post-translationally cleaved by carboxypeptidase etc., and the like. Moreover, in the present disclosure, the anti-EphA4 antibody where the C-terminal lysine of the heavy chain is deleted includes not only an anti-EphA4 antibody where the C-terminal lysine is deleted in both heavy chains, but also an anti-EphA4 antibody where the C-terminal lysine is deleted in only one heavy chain.

In one aspect, the present disclosure relates to an isolated nucleic acid encoding an anti-EphA4 antibody. An isolated nucleic acid encoding an anti-EphA4 antibody refers to one or more nucleic acid molecules encoding the heavy chain and/or light chain of an anti-EphA4 antibody. In one embodiment, the nucleic acid according to the present disclosure encodes the heavy chain of an anti-EphA4 antibody. In another embodiment, the nucleic acid according to the present disclosure encodes the light chain of an anti-EphA4 antibody. In yet another embodiment, the nucleic acid according to the present disclosure encodes the heavy chain and light chain of an anti-EphA4 antibody. The nucleic acid according to the present disclosure also includes a first nucleic acid molecule encoding the heavy chain of an anti-EphA4 antibody and a second nucleic acid molecule encoding the light chain of an anti-EphA4 antibody.

In another aspect, the present disclosure relates to a vector comprising an isolated nucleic acid encoding an anti-EphA4 antibody. The vector according to the present disclosure refers to one or more vectors comprising an isolated nucleic acid encoding an anti-EphA4 antibody. In one embodiment, the vector according to the present disclosure is a vector comprising the nucleic acid encoding the heavy chain of an anti-EphA4 antibody and the nucleic acid encoding the light chain of an anti-EphA4 antibody. In another embodiment, the vector according to the present disclosure is a vector comprising the nucleic acid encoding the heavy chain and light chain of an anti-EphA4 antibody. In yet another embodiment, the vector according to the present disclosure comprises a fist vector comprising the nucleic acid encoding the heavy chain of an anti-EphA4 antibody and a second vector comprising the nucleic acid encoding the light chain of an anti-EphA4 antibody. The vector according to the present disclosure may be, but is not particularly limited to, a plasmid, a cosmid, a virus, a phage, and the like. For example, as a viral vector, a retrovirus, a lentivirus, an adenovirus, an adeno-associated virus, or a herpes simplex virus vector, and the like are also included in the vector according to the present disclosure.

In yet another aspect, also included in the present disclosure are a host cell comprising the vector according to the present disclosure, and a method of generating an anti-EphA4 antibody comprising a step of culturing the aforementioned host cell. The host cell according to the present disclosure may be, but is not particularly limited to, an E. coli cell, monkey COS cell, a Chinese hamster ovary (CHO) cell, an NS0 cell, and the like. In one embodiment, the method of generating an anti-EphA4 antibody comprises the step of culturing the host cell and a step of collecting the anti-EphA4 antibody secreted from the aforementioned host cell (or the culture medium of the host cell).

The anti-EphA4 antibody according to the present disclosure is used for treating ALS. Accordingly, the present disclosure relates to a pharmaceutical composition for treating ALS comprising the anti-EphA4 antibody according to the present disclosure. In another aspect, the present disclosure also encompasses an ALS therapeutic method comprising administering a therapeutically effective amount of an anti-EphA4 antibody to a subject suffering from ALS.

In yet another aspect, the present disclosure also encompasses the use of anti-EphA4 antibody for manufacturing a therapeutic drug for ALS.

In yet another aspect, the present disclosure also encompasses an anti-EphA4 antibody for use in treating ALS.

The anti-EphA4 antibody according to the present disclosure can be used alone, or in combination with other agents or compositions in the therapeutic method. For example, the anti-EphA4 antibody according to the present disclosure may be administered at the same or different times as another agent. Such combination therapy includes combination administration (two or more agents are contained in the same or separate formulation) and separate administration (such as simultaneous or sequential). When two or more agents are administered separately, the administration of the anti-EphA4 antibody according to the present disclosure may be carried out preceding or following the concomitant therapeutic method.

The subject to which the anti-EphA4 antibody according to the present disclosure is administered is not limited, and for example the present invention can be employed on human or non-human mammals (such as monkey, mouse, rat, rabbit, cow, horse, goat).

The administration method (administration route, dosage, number of administrations per day, administration timing, and the like) of the anti-EphA4 antibody according to the present disclosure to the subject is not particularly limited, and it can be appropriately determined by those skilled in the art (such as a physician) depending on the health state of the subject, the extent of the disease, the type of agents used in combination, and the like.

The pharmaceutical composition according to the present disclosure comprises the above anti-EphA4 antibody according to the present disclosure. The pharmaceutical composition according to the present disclosure can be e.g. manufactured according to known methods such as methods described in Pharmacopeia of Japan (JP), United States Pharmacopeia (USP), or European Pharmacopeia (EP).

Those skilled in the art will recognize that as long as it is not technically contradicting, any one or more of any and all aspects described herein may be appropriately combined to carry out the present disclosure. Further, those skilled in the art will recognize that as long as it is not technically contradicting, it will be preferred that any and all preferred or advantageous aspects described herein are appropriately combined to carry out the present disclosure.

All of the disclosures of the literatures cited herein should be deemed as clearly cited herein by reference, and those skilled in the art can cite and recognize the related disclosed contents in these literatures as a part of the present specification according to the context herein without departing from the spirit and scope of the present disclosure.

The literatures cited herein are provided solely for the purpose of disclosing related technology preceding the filing date of the present application, and are not to be construed as an admission by the present inventors that the present invention does not hold the right to precede said disclosures due to prior inventions or for any other reason. All descriptions in these literatures are based on the information available to the present applicants, and do not configure in any way that the contents of these descriptions are accurate.

The terms used herein are employed for describing particular embodiments, and do not intend to limit the invention.

The term "comprise" employed herein, unless the content clearly indicates to be understood otherwise, intends the presence of the described items (such as components, steps, elements, or numbers), and does not exclude the presence of other items (such as components, steps, elements, and numbers). The term "consist of" encompasses the aspects described with the terms "consist of" and/or "consist essentially of."

The term "neutralizing activity" employed herein means the activity to inhibit the binding between EphA4 and its ligand, and/or the activity to inhibit signal transduction and molecular expression response or functionality change of cells induced by EphA4 by binding to its ligand in a living human body.

Unless otherwise defined, all terms used herein (including technical and scientific terms) have the same meaning as that broadly recognized by those skilled in the art of the technology to which the present disclosure belongs. The terms used herein, unless explicitly defined otherwise, should be construed as having meanings consistent with the meanings herein and in related technical fields, and shall not be construed as having idealized or excessively formal meanings.

Terms such as first and second are employed to express various elements, and it should be recognized that these elements are not to be limited by these terms per se. These terms are employed solely for the purpose of discriminating one element from another, and for example, it is possible to describe a first element as a second element, and similarly, to describe a second element as a first element without departing from the scope of the present disclosure.

The numeric values employed herein for indicating a component content or a numeric value range and the like, unless explicitly indicated, are to be understood as being modified by the term "about." For example, "4°C," unless explicitly indicated, is recognized as meaning "about 4°C," and it is natural that those skilled in the art can reasonably recognize the extent thereof according to technical common sense and the meaning of the present specification.

Unless clearly indicated to mean otherwise in context, when used in the specification and claims herein, it should be recognized that each aspect represented in singular form may also be a plural form as long as it is not technically contradicting, and vice versa.

The present disclosure will now be described in further detail with reference to Examples. However, the present disclosure can be embodied by various aspects, and shall not be construed as being limited to the Examples described herein. Those skilled in the art of related technical fields can carry out the present disclosure without changing the spirit and scope of the present disclosure with various modifications, additions, deletions, substitution, and the like.

### Examples

### Reference Example 1: Generation of anti-EphA4 monoclonal antibody

### (A) Generation of mouse anti-EphA4 monoclonal antibody

In order to generate a monoclonal antibody that binds to a mouse EphA4 (Genbank Accession No. NP_031962.2, SEQ ID NO. 1), a protein having secretory alkaline phosphatase (SEAP) and a histidine tag fused to the extracellular region of mouse EphA4 (positions 20-547) (SEQ ID NO. 2) (hereinafter referred to as "mouse EphA4 extracellular region-SEAP-His protein," SEQ ID NO. 3) was prepared by the following steps.

First, a DNA sequence encoding the signal sequence (SEQ ID NO. 4) and the extracellular region (SEQ ID NO. 2) of mouse EphA4 was amplified by RT-PCR employing total RNA derived from mouse brain, and cloned into the SalI/NotI site of a pENTRlA vector (Invitrogen/Life Technologies) having a DNA sequence encoding SEAP and a histidine tag. Next, a DNA sequence encoding the signal sequence and the extracellular region of mouse EphA4, SEAP, and a histidine tag was transferred to a pcDNA 3.1_rfcB vector by the LR reaction of the Gateway System (Invitrogen/Life Technologies), and pcDNA 3.1-mouse EphA4 extracellular region-SEAP-His expression vector was constructed. The constructed pcDNA 3.1-mouse EphA4 extracellular region-SEAP-His expression vector was transfected into HEK293EBNA cells (Invitrogen/Life Technologies) with TransIT-LT1 (TAKARA) . After 6 days of incubation (5% CO₂, 37°C), the culture supernatant was collected. From the collected culture supernatant, mouse EphA4 extracellular region-SEAP-His protein (SEQ ID NO. 3) was purified with a Protino column (MACHEREY-NAGEL).

Twenty micrograms of mouse EphA4 extracellular region-SEAP-His protein was mixed with the same amount of TiterMax Gold adjuvant (TiterMax USA) or GERBU adjuvant (GERBU Biotechnik GmbH), and subcutaneously injected in the footpad of a Balb/c mouse. Mouse EphA4 extracellular region-SEAP-His protein was then similarly administered on Days 3, 7, and 10. In doing so, TiterMax Gold adjuvant (TiterMax USA) was used only on Day 10, and GERBU adjuvant (GERBU Biotechnik GmbH) was used on Days 3, 7, and 10. The mouse was sacrificed on Day 13, and peripheral lymph nodes were collected to prepare lymph node cells. In the presence of GenomeONE-CF (Ishihara Sangyo Kaisha, Ltd.), the prepared lymph node cells and P3U1 myeloma cells (endowed from Kyoto University) were fused at a proportion of 5:1. The fused cells were cultured in a 96-well plastic plate. After 7 days of incubation (5% CO₂, 37°C), the culture supernatant was collected.

The culture supernatant obtained was employed to pick up wells having reactivity against mouse, rat, and human EphA4.

The reactivity against mouse, rat, and human EphA4 was evaluated with ELISA employing a protein having the Fc region of human IgG₁ and a histidine tag fused to the extracellular region of mouse EphA4, the extracellular region (positions 20-547) of rat EphA4 (Genbank Accession No. NP_001155883.1), or the extracellular region (positions 20-547) (SEQ ID NO. 6) of human EphA4 (Genbank Accession No. NP_004429.1, SEQ ID NO. 5) (hereinafter referred to as "mouse EphA4 extracellular region-Fc-His protein," "rat EphA4 extracellular region-Fc-His protein," or "human EphA4 extracellular region-Fc-His protein," respectively).

The mouse, rat, or human EphA4 extracellular region-Fc-His protein was prepared by the following steps. To start, pcDNA 3.1-mouse, rat, or human EphA4 extracellular region-Fc-His expression vector was constructed. First, a DNA sequence encoding the signal sequence and the extracellular region of mouse, rat, or human EphA4 was amplified by RT-PCR employing total RNA derived from mouse, rat, or human brain, and cloned into the SalI/NotI site of a pENTRlA vector (Invitrogen/Life Technologies) having a DNA sequence encoding Fc and a histidine tag. Next, a DNA sequence encoding the signal sequence and the extracellular region of mouse, rat, or human EphA4, Fc, and a histidine tag was transferred to a pcDNA 3.1_rfcB vector by the LR reaction of the Gateway System (Invitrogen/Life Technologies), and pcDNA 3.1-mouse, rat, or human EphA4 extracellular region-Fc-His expression vector was constructed. These constructed expression vectors were transfected into HEK293EBNA cells (Invitrogen/Life Technologies) with TransIT-LT1 (TAKARA). After 6 days of incubation (5% CO₂, 37°C), the culture supernatant was collected.

ELISA employing the mouse, rat, or human EphA4 extracellular region-Fc-His protein was carried out according to the following steps. Anti-human IgG antibodies (Jackson ImmunoResearch Laboratories) were coated onto the wells of a 96-well plate (Nunc). After incubating at 4°C overnight, the wells were blocked at room temperature for 1 hour by 1 x Block Ace (Dainippon Pharmaceutical) . After washing 3 times with 0.02% Tween 20/PBS (Nacalai Tesque), the culture supernatant comprising the mouse, rat, or human EphA4 extracellular region-Fc-His protein was added to each well (final concentration 1 nM), and incubated at room temperature for 1 hour. After washing 3 times, the culture supernatant of the fused cells was supplemented to each well. After incubating at room temperature for 1 hour and washing 3 times, horseradish peroxidase-labeled anti-mouse IgG antibody (Jackson ImmunoResearch Laboratories) was added, and incubated at room temperature for 1 hour. After washing 3 times, TMBZ (3,3',5,5'-tetramethylbenzidine, Sigma) solution was added to each well, and incubated at room temperature for 5-20 minutes. An equal amount of quenching solution (2N H₂SO₄, Wako Pure Chemicals) was added to each well, and absorbance at 450 nm was read by a microplate reader (PerkinElmer).

Hybridomas were cloned from wells picked up after going through the above steps with limiting dilution method, ultimately yielding hybridoma clones that express mouse anti-EphA4 antibodies having binding activity against mouse, rat, and human EphA4.

The hybridoma clones obtained were cultured, and mouse anti-EphA4 monoclonal antibodies were purified from the culture supernatant employing Protein A (GE Healthcare).

### (B) Evaluation of EphA4 cleavage enhancing activity

Preparation of rat hippocampus neurons was carried out according to the following steps. Fetuses were removed from a rat on Day 18 of pregnancy (Charles River Laboratories Japan), and the heads were incised to remove the brain. After cutting out the hippocampus region under a stereomicroscope, they were placed in a digestion solution (137 mM NaCl (Wako Pure Chemicals), 5 mM KCl (Wako Pure Chemicals), 7 mM Na₂HPO₄ (Wako Pure Chemicals),25 mM Hepes (DOJINDO), 0.5 mg/mL DNase (Sigma), 0.25% trypsin (Life technologies)) and shaken at 37°C for 10 minutes. The solution was removed, and 20% Fetal bovine serum/Hanks buffer (Sigma) was added. The liquid was removed, this was washed twice with Hanks buffer, and then the hippocampus tissue was pipetted in Hanks buffer to generate a cell suspension. The cells were seeded in a 96-well dish (Falcon) coated with poly L-lysine filled with a culture medium (Neurobasal medium (Life technologies), 1 × B-27 supplement (Life technologies), 0.5 mM L-glutamine (Life technologies)).

The evaluation of EphA4 cleavage enhancing activity employing hippocampus neurons was carried out according to the following steps. Rat hippocampus neurons seeded in a 96-well dish (Falcon) were treated with anti-EphA4 monoclonal antibodies (67 nM) and γ-secretase inhibitory drug Compound E (50 nM, Enzo Life Sciences), 16 hours thereafter washed with PBS (Wako Pure Chemicals), SDS sample buffer (Laemmli sample buffer (Bio-Rad) and 5% 2-mercaptoethanol (Bio-Rad)) was added and the cells were collected, and this was boiled for 5 minutes. SDS-PAGE was carried out with this sample, western blotting employing anti-EphA4 monoclonal antibodies (Abnova) was carried out, the band strength was quantified, and the value of EphA4 C-terminal fragment/full length EphA4 was calculated.

A mouse anti-EphA4 monoclonal antibody (antibody A) having the activity to enhance the cleavage of EphA4 was obtained. The isotype of antibody A was determined by a monoclonal antibody isotyping kit (Serotec) to be IgG₁ for the heavy chain an κ for the light chain.

### (C) Sequence analysis of antibody A

A DNA sequence encoding the signal sequences of the heavy and light chains of antibody A as well as the variable region was amplified by the 5'-RACE (5'-rapid amplification of cDNA ends) method. From the hybridoma, total RNA was prepared with RNeasy (QIAGEN), and treated with DNase (QIAGEN, RNase free DNase set). A double-stranded cDNA was prepared from the total RNA employing a cDNA synthesis kit (TAKARA). A 5' adaptor obtained from annealing of oligo DNA ad29S (ACATCACTCCGT) (SEQ ID NO. 7) and oligo DNA ad29AS (ACGGAGTGATGTCCGTCGACGTATCTCTGCGTTGATACTTCAGCGTAGCT) (SEQ ID NO. 8) was added to the cDNA. The cDNA obtained was amplified with a 5' forward primer (5'-PCR4 primer, AGCTACGCTGAAGTATCAACGCAGAG) (SEQ ID NO. 9) and a 3' reverse primer (GCCAGTGGATAGACTGATGG (SEQ ID NO. 10) was employed for amplification of the mouse IgG heavy chain and GATGGATACAGTTGGTGCAGC (SEQ ID NO. 11) was employed for amplification of mouse Igκ light chain). The cDNA amplified was inserted into a pCR2.1 vector (Invitrogen/Life Technologies) . The gene sequence of antibody A was analyzed with ABI3130XL. As amino acid sequences encoded by the gene sequence of antibody A identified by this analysis, the heavy chain signal sequence is the sequence shown in SEQ ID NO. 12, the heavy chain variable region is the sequence shown in SEQ ID NO. 13, the light chain signal sequence is the sequence shown in SEQ ID NO. 14, and the light chain variable region is the sequence shown in SEQ ID NO. 15. As nucleotide sequences encoding the gene sequence of antibody A, the heavy chain signal sequence is the sequence shown in SEQ ID NO. 16, the heavy chain variable region is the sequence shown in SEQ ID NO. 17, the light chain signal sequence is the sequence shown in SEQ ID NO. 18, and the light chain variable region is the sequence shown in SEQ ID NO. 19.

The full-length sequences of the heavy and light chains of antibody A were obtained by the following steps. From the hybridoma, total RNA was prepared with RNeasy (QIAGEN), and treated with DNase (QIAGEN, RNase free DNase set) . A reverse transcription product was prepared from the total RNA employing an RNA PCR kit (TAKARA) . With the reverse transcription product obtained as the template, the gene sequences encoding the heavy and light chains of antibody A were amplified by PCR employing a 5' forward primer (GCGAAGCTTGCCGCCACCATGGCTGTCCTGGTGCTGCTCC (primer ID 7455) (SEQ ID NO. 20) was used for amplification of the heavy chain and GCGAAGCTTGCCGCCACCATGGACATGAGGGTTCCTGCTCACG (primer ID 7453) (SEQ ID NO. 21) was used for amplification of the light chain) and a 3' reverse primer (GCGGAATTCATCATTTACCAGGAGAGTGGGAGAGGC (primer ID 7257) (SEQ ID NO. 22) was used for amplification of the heavy chain and CGCGAATTCACTAACACTCATTCCTGTTGAAGCTCTTGAC (primer ID 7249) (SEQ ID NO. 23) was used for amplification of the light chain), and respectively cloned into pEE6.4 and pEE12.4 vectors (Lonza). The gene sequence was analyzed with ABI3130XL. As amino acid sequences encoded by the gene sequence of antibody A identified by this analysis, the heavy chain constant region is the sequence shown in SEQ ID NO. 24 and the light chain constant region is the sequence shown in SEQ ID NO. 25.

The CDRs of antibody A were determined with the following method. The amino acid sequence of antibody A was numbered with Abysis software (UCL) according to the Kabat numbering system. Based on this number, determination was made according to the Kabat definition for identification of CDR. The amino acid sequence of the CDRs of antibody A is shown in Table 1.

**[Table 1]**

| Amino acid sequence of CDRs of antibody A | |
|---|---|
| Name | Sequence |
| Heavy chain CDR1 | RYGVH (SEQ ID NO. 26) |
| Heavy chain CDR2 | VIWRGGSTDYNAAFMS (SEQ ID NO. 27) |
| Heavy chain CDR3 | ESLFGVYYDYGYYSMDY (SEQ ID NO. 28) |
| Light chain CDR1 | RASQEISGYLS (SEQ ID NO. 29) |
| Light chain CDR2 | AASTLDS (SEQ ID NO. 30) |
| Light chain CDR3 | LQYASYPLT (SEQ ID NO. 31) |

### Reference Example 2: Binding affinity of anti-EphA4 monoclonal antibody against mouse and human EphA4

The binding affinity of antibody A against mouse and human EphA4 was determined by surface plasmon resonance (SPR method) employing Biacore T200 (GE Healthcare). First, anti-His antibodies (GE Healthcare, 28-9950-56) were fixed to a sensor chip CM5. Fixation was carried out by amine coupling method employing N-hydroxysuccinimide (NHS) and N-ethyl-N'-(3-dimethylaminopropyl) carbodiimide hydrochloride salt (EDC), and ethanolamine was employed for blocking (the sensor chip and reagents for fixation are all from GE Healthcare) . This was diluted with the fixation buffer (10 mM sodium acetate, pH 4.5) to 3.5 µg/mL, and fixed onto the sensor chip according to the protocol accompanying Biacore T200. Mouse or human EphA4 extracellular region-SEAP-His 10 was diluted with the running buffer HBS-EP (GE Healthcare, BR-1001-88), and the solution was sent and captured onto the flow cell for 120 seconds (about 10 RU of captured amount). Subsequently, antibody A serially diluted to a range of 100, 50, 25, 12.5, 6.3, 3.2, 1.6, 0 nM with HBS-EP was added to the sensor chip for 120 seconds, and the binding reaction curve at the time of addition (binding phase, 120 seconds) and after completing the addition (dissociation phase, 600 seconds) was sequentially observed. Upon completion of each observation, 4 M MgCl₂ (60 seconds, Wako Pure Chemicals) was added to regenerate the sensor chip. Fitting analysis by 1:1 binding model employing the software accompanying the system, BIA evaluation software, was carried out against the binding reaction curve obtained, and the binding affinity (KD = kd/ka) against mouse and human EphA4 was calculated.

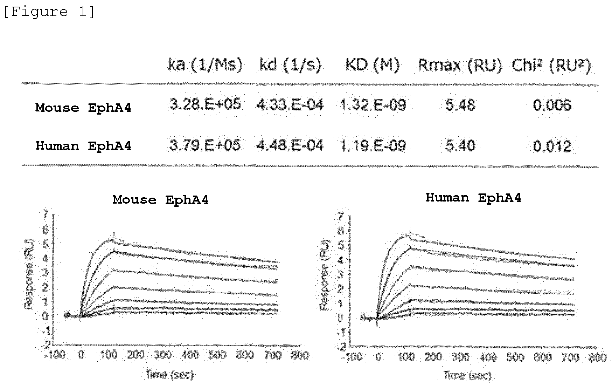

The binding affinity of antibody A against mouse and human EphA4 (KD value) was 1.32 × 10⁻⁹ M and 1.19 × 10⁻⁹ M, respectively (Figure 1). Other binding parameters against mouse and human EphA4 were almost similar. Accordingly, it is thought that antibody A has similar binding affinity against mouse and human EphA4.

### Reference Example 3: EphA4 cleavage enhancing activity of anti-EphA4 monoclonal antibody in hippocampus neurons

For antibody A, the evaluation of EphA4 cleavage enhancing activity employing hippocampus neurons was carried out according to the following steps. Rat hippocampus neurons seeded in a 96-well dish (Falcon) were treated with antibody A (2.0, 6.7, 20 nM) and γ-secretase inhibitory drug Compound E (50 nM, Enzo Life Sciences), 24 hours thereafter washed with PBS (Wako Pure Chemicals), SDS sample buffer (Laemmli sample buffer (Bio-Rad) and 5% 2-mercaptoethanol (Bio-Rad)) was added and the cells were collected, and this was boiled for 5 minutes. SDS-PAGE was carried out with this sample, western blotting employing anti-EphA4 monoclonal antibodies (Abnova) was carried out, the band strength was quantified, and the value of EphA4 C-terminal fragment/full length EphA4 was calculated.

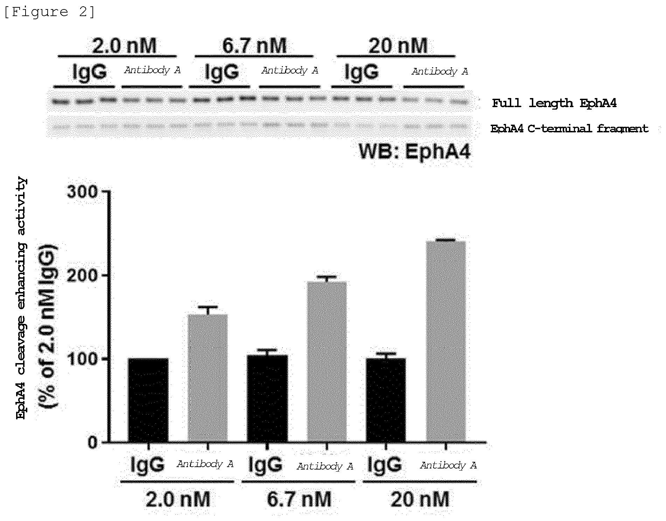

Antibody A concentration-dependently enhanced EphA4 cleavage reaction in hippocampus neurons (Figure 2).

### Reference Example 4: Mouse EphA4-mouse ligand binding inhibitory activity of anti-EphA4 monoclonal antibody

For antibody A, the evaluation of binding inhibitory activity between mouse EphA4 and mouse ligand was carried out according to the following steps. Anti-alkaline phosphatase antibodies (Thermo SCIENTIFIC) were coated onto the wells of a 96-well plate (Nunc). After incubating at 4°C overnight, the wells were blocked at room temperature for 1 hour by 1% Block Ace (DS Pharma Biomedical) . After washing 3 times with 0.02% Tween 20/PBS (Thermo SCIENTIFIC), mouse EphA4 extracellular region-SEAP-His protein was added to the wells (final concentration 10 nM), and incubated at room temperature for 1 hour. After washing 3 times, the ligand and antibody A (0, 0.003, 0.01, 0.03, 0.1, 0.3, 1, 3, 10, 30, 100, 300, 1000, 3000 nM) were supplemented to the wells. Note that biotinylated mouse EphrinA1-Fc chimera (R&D Systems, final concentration 6 nM) and biotinylated mouse EphrinB2-Fc chimera (R&D Systems, final concentration 2.5 nM) were employed as ligands. After incubating at room temperature for 1 hour and washing 3 times, horseradish peroxidase-labeled streptavidin (GE Healthcare) was added, and incubated at room temperature for 1 hour. After washing 3 times, TMBZ (3,3',5,5'-tetramethylbenzidine, Sigma) solution was added to the wells, and incubated at room temperature for 2 minutes. An equal amount of quenching solution (1 N H₂SO₄, Wako Pure Chemicals) was added to the wells, and absorbance at 450 nm was read by a microplate reader (PerkinElmer).

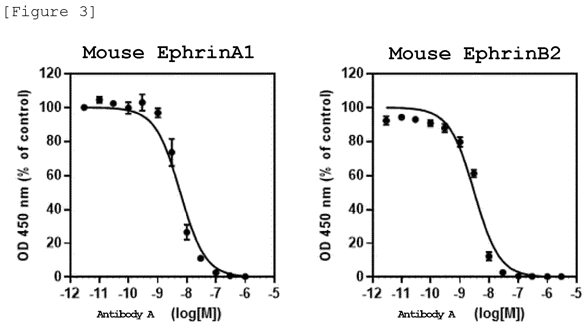

Antibody A concentration-dependently suppressed the binding between mouse EphA4 and mouse ligand, and the IC₅₀ values against mouse EphrinA1 and EphrinB2 binding were about 5.9 nM and 3.1 nM, respectively (Figure 3). Accordingly, it was shown that antibody A strongly inhibits the binding between mouse EphA4 and mouse ligand.

### Reference Example 5: Human EphA4-human ligand binding inhibitory activity of anti-EphA4 monoclonal antibody

For antibody A, the evaluation of binding inhibitory activity between human EphA4 and human ligand was carried out according to the following steps. Anti-alkaline phosphatase antibodies (Thermo SCIENTIFIC) were coated onto the wells of a 96-well plate (Nunc). After incubating at 4°C overnight, the wells were blocked at room temperature for 1 hour by 1% Block Ace (DS Pharma Biomedical) . After washing 3 times with 0.05% Tween 20/PBS (Thermo SCIENTIFIC), human EphA4 extracellular region-SEAP-His protein was added to the wells (final concentration 10 nM), and incubated at room temperature for 1 hour. After washing 3 times, the ligand and serially diluted antibody A (0, 0.003, 0.01, 0.03, 0.1, 0.3, 1, 3, 10, 30, 100, 300, 1000, 3000 nM) were supplemented to the wells. Note that biotinylated human EphrinA5-Fc chimera (R&D Systems, final concentration 0.7 nM) and biotinylated human EphrinB3-Fc chimera (R&D Systems, final concentration 2.3 nM) were employed as ligands. After incubating at room temperature for 1 hour and washing 3 times, horseradish peroxidase-labeled streptavidin (GE Healthcare) was added, and incubated at room temperature for 1 hour. After washing 3 times, TMBZ (3,3',5,5'-tetramethylbenzidine, Sigma) solution was added to the wells, and incubated at room temperature for 2-5 minutes. An equal amount of quenching solution (1 N H₂SO₄, Wako Pure Chemicals) was added to the wells, and absorbance at 450 nm was read by a microplate reader (Molecular Devices or PerkinElmer).

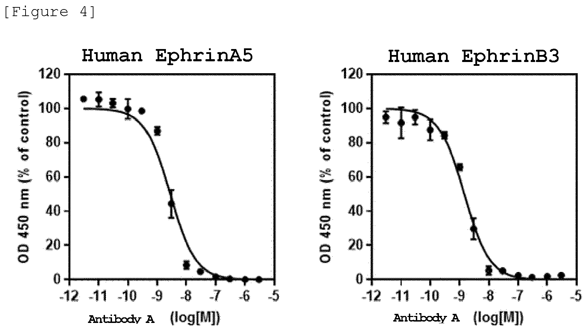

Antibody A concentration-dependently suppressed the binding between human EphA4 and human ligand, and the IC₅₀ values against human EphrinA5 and EphrinB3 binding were about 2.8 nM and 1.4 nM, respectively (Figure 4). Accordingly, it was shown that antibody A also strongly inhibits the binding between human EphA4 and human ligand.

### Reference Example 6: Selectivity of anti-EphA4 monoclonal antibody against human Eph receptor

According to the method for preparing the mouse EphA4 extracellular region-SEAP-His protein described in Reference Example 1, DNA sequences encoding the signal sequence and the extracellular region of each Eph receptor (EphA1, EphA2, EphA3, EphA4, EphA5, EphA6, EphA7, EphA8, EphA10, EphB1, EphB2, EphB3, EphB4, EphB6) of human were amplified by RT-PCR employing total RNA derived from tissue, and cloned into a pENTRlA vector (Invitrogen/Life Technologies) having a DNA sequence encoding SEAP and a histidine tag. Next, a DNA sequence encoding the signal sequence and the extracellular region of each Eph receptor of human, SEAP, and a histidine tag was transferred to a pcDNA 3.1_rfcB vector by the LR reaction of the Gateway System (Invitrogen/Life Technologies), and a vector expressing a protein having SEAP and a His tag fused to the extracellular region of each Eph receptor of human (referred to as "Eph receptor extracellular region-SEAP-His protein") (referred to as "Eph receptor extracellular region-SEAP-His protein expression vector") was constructed.

Next, employing the Expi293 expression system (Gibco/Thermo Fisher), expression vectors for each Eph receptor of human extracellular region-SEAP-His protein were introduced into Expi293F cells (Gibco/Thermo Fisher) . After 5 days of culture (5% CO2, 37°C, 120 rpm), the culture supernatant was collected, and centrifuged at room temperature at 1500 rpm for 5 minutes. The centrifugation supernatant was filtered with a 0.45 µm filter (Millipore).

For antibody A, the evaluation of the binding activity of human Eph receptor was carried out according to the following steps.

Rabbit anti-6-His antibodies (Bethyl Laboratories) were coated onto the wells of a 96-well plate (Nunc). After incubating at 4°C overnight, the wells were blocked at room temperature for 1 hour by 1% Block Ace (DS Pharma Biomedical) . After washing 3 times with 0.05% Tween 20/PBS (Thermo SCIENTIFIC), each Eph receptor of human extracellular region-SEAP-His protein (final concentration 1 nM) was seeded into each well, and incubated at room temperature for 1 hour. After washing 3 times, human IgG solution (100 µg/mL, Mitsubishi Pharma Corporation) and antibody A (10 µg/mL) were supplemented to the wells, and incubated at room temperature for 1 hour. Horseradish peroxidase-labeled donkey anti-mouse IgG antibody (Jackson ImmunoResearch Laboratories) was added, and incubated at room temperature for 1 hour. After washing 3 times, TMBZ (3,3',5,5'-tetramethylbenzidine, Sigma) solution was added to the wells, and upon confirmation of modest coloring, an equal amount of quenching solution (1 N H₂SO₄, Wako Pure Chemicals) was added to the wells, and absorbance at 450 nm was read by a microplate reader (PerkinElmer).

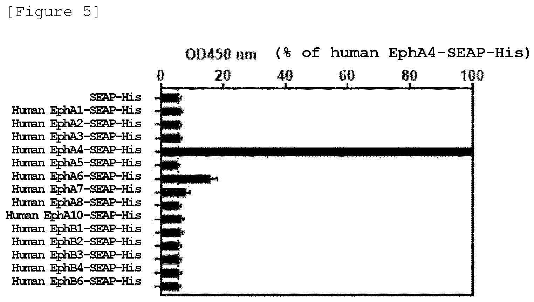

Antibody A had specific binding activity only to human EphA4 among the human Eph receptor family (Figure 5).

### Reference Example 7: selectivity of anti-EphA4 monoclonal antibody against mouse Eph receptor

According to the method for preparing the EphA4 extracellular region-Fc-His protein described in Reference Example 1, DNA sequences encoding the signal sequence and the extracellular region of each Eph receptor (EphA1, EphA3, EphA4, EphA5, EphA6, EphA7, EphA8, EphA10, EphB1, EphB2, EphB3, EphB4, EphB6) of mouse were amplified by RT-PCR employing total RNA derived from tissue, and cloned into a pENTRlA vector having a DNA sequence encoding the Fc region of human IgG₁ and a histidine tag (Invitrogen/Life Technologies). Next, a DNA sequence encoding the signal sequence and the extracellular region of each Eph receptor (EphA1, EphA3, EphA4, EphA5, EphA6, EphA7, EphA8, EphA10, EphB1, EphB2, EphB3, EphB4, EphB6) of mouse, Fc, and a histidine tag was transferred to a pcDNA 3.1_rfcB vector by the LR reaction of the Gateway System (Invitrogen/Life Technologies), and expression vectors for each Eph receptor of mouse extracellular region-Fc-His protein were constructed. For construction of mouse EphA2 extracellular region-Fc-His protein expression vector, a DNA sequence encoding the signal sequence and the extracellular region of mouse EphA2 was amplified by RT-PCR employing total RNA derived from tissue, cloned into a pcDNA 3.1 vector having a DNA sequence encoding Fc and a histidine tag, and mouse EphA2 extracellular region-Fc-His protein expression vector was constructed.

Next, employing the Expi293 expression system (Gibco/Thermo Fisher), expression vectors for each Eph receptor of mouse extracellular region-Fc-His protein were introduced into Expi293F cells (Gibco/Thermo Fisher) . After 5 days of culture (5% CO2, 37°C, 120 rpm), the culture supernatant was collected, and centrifuged at room temperature at 1500 rpm for 5 minutes. The centrifugation supernatant was filtered with a 0.45 µm filter (Millipore).

For antibody A, the evaluation of the binding activity of mouse Eph receptor was carried out according to the following steps.

Rabbit anti-6-His antibodies (Bethyl Laboratories) were coated onto the wells of a 96-well plate (Nunc). After incubating at 4°C overnight, the wells were blocked at room temperature for 1 hour by 1% Block Ace (DS Pharma Biomedical) . After washing 3 times with 0.05% Tween 20/PBS (Thermo SCIENTIFIC), each Eph receptor of mouse extracellular region-Fc-His protein (final concentration 1 nM) was seeded into each well, and incubated at room temperature for 1 hour. After washing 3 times, human IgG solution (100 µg/mL, Sigma) and antibody A (10 µg/mL) were supplemented to the wells, and incubated at room temperature for 1 hour. Horseradish peroxidase-labeled donkey anti-mouse IgG antibody (Jackson ImmunoResearch Laboratories) was added, and incubated at room temperature for 1 hour. After washing 3 times, TMBZ (3,3',5,5'-tetramethylbenzidine, Sigma) solution was added to the wells, and upon confirmation of modest coloring, an equal amount of quenching solution (1 N H₂SO₄, Wako Pure Chemicals) was added to the wells, and absorbance at 450 nm was read by a microplate reader (PerkinElmer).

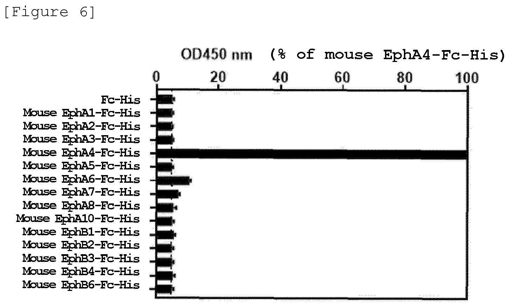

Antibody A had specific binding activity only to mouse EphA4 among the mouse Eph receptor family (Figure 6).

### Reference Example 8: Reactivity of anti-EphA4 monoclonal antibody against mouse, rat, monkey, and human EphA4

Generation of mouse, rat, monkey, and human EphA4 extracellular region-Fc-His proteins was carried out according to the following steps. First, according to the method for preparing the EphA4 extracellular region-Fc-His protein described in Reference Example 1, monkey EphA4 extracellular region-Fc-His protein expression vector was constructed. The amino acid sequence of monkey EphA4 utilized in the vector construction is shown as SEQ ID NO. 32, and the extracellular region thereof is shown as SEQ ID NO. 33. Employing monkey EphA4 extracellular region-Fc-His protein expression vector, as well as mouse, rat, and human EphA4 extracellular region-Fc-His protein expression vectors described in Reference Example 1, various EphA4 extracellular region-Fc-His protein were prepared.

For antibody A, the evaluation of the binding activity with various EphA4 extracellular regions was carried out according to the following steps.

Donkey anti-human IgG antibodies (Jackson ImmunoResearch Laboratories) were coated onto the wells of a 96-well plate (Nunc). After incubating at 4°C overnight, the wells were blocked at room temperature for 1 hour by 1% Block Ace (DS Pharma Biomedical) . After washing 3 times with 0.05% Tween 20/PBS (Thermo SCIENTIFIC), mouse, rat, monkey, and human EphA4 extracellular region-Fc-His proteins (final concentration 1 nM) were seeded into the wells, and incubated at room temperature for 1 hour. After washing 3 times, human IgG solution (100 µg/mL, Mitsubishi Pharma Corporation) and antibody A (0, 0.00013, 0.00064, 0.0032, 0.016, 0.08, 0.4, 2, 10, µg/mL) were supplemented to the wells, and incubated at room temperature for 1 hour. Horseradish peroxidase-labeled donkey anti-mouse IgG antibody (Jackson ImmunoResearch Laboratories) was added, and incubated at room temperature for 1 hour. After washing 3 times, TMBZ (3,3',5,5'-tetramethylbenzidine, Sigma) solution was added to the wells, and upon confirmation of modest coloring, an equal amount of quenching solution (1 N H₂SO₄, Wako Pure Chemicals) was added to the wells, and absorbance at 450 nm was read by a microplate reader (PerkinElmer).

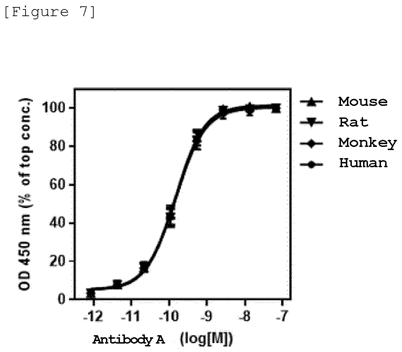

Antibody A had equivalent binding activity in any of mouse, rat, monkey, and human EphA4 (Figure 7).

### Reference Example 9: Reactivity of anti-EphA4 monoclonal antibody against human EphA4 extracellular region, ligand binding domain, fibronectin type III domain 1, and fibronectin type III domain 2

The generation of a protein having the extracellular region (ECD), ligand binding domain (LBD), fibronectin type III domain 1 (FN1), or fibronectin type III domain 2 (FN2) of human EphA4 fused to a maltose-binding protein (MBP) and a histidine tag (hereinafter referred to as "human EphA4 extracellular region-MBP-His protein," "human EphA4 ligand binding domain-MBP-His protein," "human EphA4 fibronectin type III domain 1-MBP-His protein," and "human EphA4 fibronectin type III domain 2-MBP-His protein") was carried out according to the following steps. To start, pcDNA 3.4-human EphA4 extracellular region, ligand binding domain, fibronectin type III domain 1, or fibronectin type III domain 2-MBP-His expression vector was constructed. First, a DNA sequence encoding the signal sequence of human EphA4 (SEQ ID NO. 34) or the signal sequence of preprotrypsin (SEQ ID NO. 35) and each domain of human EphA4 was amplified by PCR, cloned into a pcDNA 3.4 vector having a DNA sequence encoding MBP and a histidine tag (Invitrogen/Life Technologies), and expression vectors for human EphA4 extracellular region-MBP-His protein, human EphA4 ligand binding domain-MBP-His protein, human EphA4 fibronectin type III domain 1-MBP-His protein, and human EphA4 fibronectin type III domain 2-MBP-His protein were constructed. The amino acid sequence of human EphA4 utilized in the vector construction is shown as SEQ ID NO. 5, the extracellular region thereof as SEQ ID NO. 36, the ligand binding domain as SEQ ID NO. 37, the fibronectin type III domain 1 as SEQ ID NO. 38, and the fibronectin type III domain 2 as SEQ ID NO. 39. Employing the Expi293 expression system (Thermo SCIENTIFIC), the above expression vector was transfected into Expi293F cells (Thermo SCIENTIFIC). After 4 days, the culture supernatant was collected, and passed through a 0.45 µm filter (Millipore). Crude purification was carried out with amylose resin (NEB), and the buffer was substituted to PBS (Wako Pure Chemicals) with Zeba Spin Desalting column (Thermo SCIENTIFIC). The monomer fraction was fraction purified with Superdex 200 10/300 (GE Healthcare).

For antibody A, the evaluation of binding activity with various domains in human EphA4 was carried out according to the following steps.

Rabbit anti-6-His antibodies (Bethyl Laboratories) were coated onto the wells of a 96-well plate (Nunc). After incubating at 4°C overnight, the wells were blocked at room temperature for 1 hour by 1% Block Ace (DS Pharma Biomedical). After washing twice with 0.02% Tween 20/PBS (Nacalai Tesque), human EphA4 extracellular region-MBP-His protein, human EphA4 ligand binding domain-MBP-His protein, human EphA4 fibronectin type III domain 1-MBP-His protein, and human EphA4 fibronectin type III domain 2-MBP-His protein (final concentration 10 nM) were seeded into the wells, and incubated at room temperature for 1 hour. After washing 3 times, antibody A (final concentration 10 nM) was supplemented to the wells, and incubated at room temperature for 1 hour. Horseradish peroxidase-labeled goat anti-mouse IgG Fcy fragment antibody (Jackson ImmunoResearch Laboratories) was added, and incubated at room temperature for 1 hour. After washing 5 times, TMB solution (KPL) was added to the wells, and upon confirmation of modest coloring, an equal amount of quenching solution (2 N H₂SO₄, Wako Pure Chemicals) was added to the wells, and absorbance at 450 nm and 650 nm were read by a microplate reader (PerkinElmer).

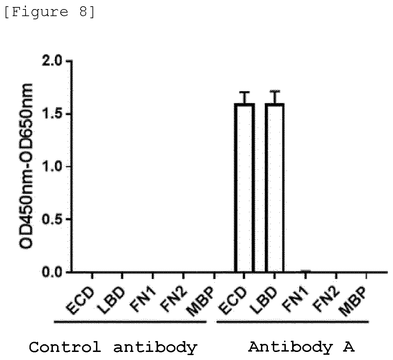

Antibody A had binding activity with human EphA4 extracellular region (ECD) and ligand binding domain (LBD) (Figure 8) . It did not react to fibronectin type III domain 1 (FN1) and fibronectin type III domain 2 (FN2) . Accordingly, it was found that antibody A specifically binds to the ligand binding domain of human EphA4 extracellular region.

### Reference Example 10: Increasing effect of anti-EphA4 monoclonal antibody against number of spines in hippocampus neuron

Preparation of rat hippocampus neurons was carried out as described in the above Reference Example 1 (B). The EGFP gene was introduced into rat hippocampus neurons with Nucleofector (Lonza), mixed with rat hippocampus neurons without gene introduction, and seeded in a 24-well plate (Falcon) containing a cover glass (Matsunami Glass Ind.,Ltd.) coated with poly L-lysine.

The counting of spine employing hippocampus neurons was carried out according to the following steps. EGFP-introduced rat hippocampus neurons at Day 13 of culture that were seeded in a 24-well plate (Falcon) containing a cover glass (Matsunami Glass Ind.,Ltd.) coated with poly L-lysine were treated with control antibody (mouse IgG1; BioLegend) or antibody A (6.7, 20 nM) for 24 hours. Then, the cover glass was transferred to 2% PFA (Wako Pure Chemicals)/4% Sucrose (Wako Pure Chemicals)/PBS, and left still for 20 minutes to fix the cells. After removing the fixing solution and washing the cells 3 times with PBS, 0.25% TritonX-100 (Wako Pure Chemicals)/PBS was added, and cell permeabilization was carried out for 15 minutes. After removing the solution, transferring the cover glass to 2% BSA (Sigma)/0.25% TritonX-100/Opti-MEM (GIBCO), and subjecting to 1 hour of blocking, anti-GFP antibody (Nacalai Tesque) was reacted for 1 hour 30 minutes. After removing the primary antibody solution and washing 3 times with PBS, the secondary antibody was reacted for 1 hour. After removing the secondary antibody solution and washing 3 times with PBS, Prolong Gold antifade reagent (Molecular probes) was added and sealed, and observation was carried out with LSM800 (ZEISS). The experiment described above was carried out 3 times, neurons were extracted from two cover glasses per experiment, the spines present on each dendrite were counted with an image analysis software Imaris^{™} (Bitplane), and the number of spines per 10 µm in each neuron was calculated.

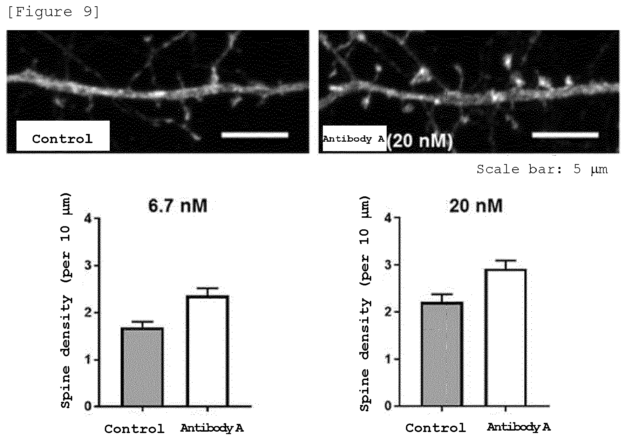

Antibody A increased the number of spines in the hippocampus neuron (Figure 9) . This result shows that antibody A has the activity to stabilize the spine in hippocampus neurons.

### Reference Example 11: Epitope mapping of EphA4-ligand binding domain (EphA4-LBD) by X-ray crystal structure analysis

Preparation of antibody A-Fab was carried out according to the following steps. Antibody A 101.1 mg was dissolved in 0.1 M sodium phosphate buffer (pH 7.0) comprising 30 mM L-cysteine and 2 mM EDTA at a concentration of 15 mg/mL. To this antibody solution, papain (Sigma) was added at an 1/200 amount to the antibody, and enzyme digestion was carried out at 37°C for 18 hours. After dialyzing the antibody A enzyme digestion juice against PBS, the precipitate was removed by centrifugation (the precipitate produced was redissolved in PBS and mixed with the centrifugation supernatant). Next, the following steps were carried out with the purpose of removing impurities other than antibody A-Fab.

### 1) Purification by Protein A column

This enzyme digestion solution was applied to a 2 mL ProSep vA High Capacity (Millipore) equilibrated with PBS, and the pass-through fraction as well as the PBS wash fraction were collected.

### 2) Affinity purification employing anti-human IgG Fcy antibody

An affinity column having anti-human IgG Fcy antibody (Jackson ImmunoResearch Laboratories) covalently bound to NHS-Activated Sepharose 4FF (GE Healthcare) was prepared according to the manual of this Sepharose. The solution collected in the above 1) was charged in this affinity column, and the pass-through solution as well as the PBS wash solution thereof were collected.

### 3) Purification by gel filtration

The pass-through fraction obtained in the above 2) was concentrated with an ultrafiltration membrane. Superose 12 (GE Healthcare) was equilibrated with PBS, and the concentrated sample was applied for separation and purification. A part of the separated and purified fraction was analyzed with SDS-PAGE, and the fraction comprising antibody A-Fab with high purity was collected and pooled. The sample purified as such was set as antibody A-Fab.

In order to generate a complex between the antibody A-Fab and the antigen EphA4-LBD, EphA4-LBD was prepared (Qin H. et al., J. Biol. Chem., 283: 29473-29484 (2008)). In order to allow EphA4-LBD at a molar ratio of about 1.5-folds against antibody A-Fab, 0.68 µmοl (200 µM, 3.4 mL) of EphA4-LBD and 0.45 µmοl (300 µM, 1.5 mL) of antibody A-Fab were mixed. Next, the mixed solution was applied to HILOAD 26/60 Superdex 75 prep grade (GE Healthcare), and eluted with the chromatography buffer (25 mM Tris/HCl (pH 7.5), 100 mM NaCl) . The fraction comprising the complex was analyzed with SDS PAGE, the fraction with high purity was collected and concentrated to about 40.8 mg/mL, and this was employed for crystallization.

The crystallization of the complex was carried out by sitting drop vapor diffusion method employing an automatic crystallization device Hydra II Plus One system (Matrix Technologies Corp., Ltd.). MRC-2 (Molecular Dimensions) was used as the plate. The composition of the reservoir solution was 100 mM HEPES (pH 7.5), 10% Polyethylene Glycol 8000, and 8% Ethylene Glycol, and crystallized droplets were generated by mixing this reservoir solution and the above complex solution at a volume ratio of 1:1. The crystallized plate generated was left still at 20°C.

Crystallization was carried out with the above condition, and crystals of space group P212121, lattice constants a = 71.0 Å, b = 84.5 Å, and c = 116.1 Å were obtained. Radiation light X-ray (1.0 Å) was irradiated to the crystals obtained to obtain diffraction data at 1.79 Å. The diffraction data was processed by HKL2000 (HKL Research Inc.), and phase determination was carried out by molecular substitution method. For molecular substitution method, the program PHASER (version 2.5.0, McCoy A. J. et al., J. Appl. Cryst. 40:658-674 (2007)) contained in CCP4 Software Suite (Collaborative computational project number 4, [CCP4] version 6.5.0, Acta Cryst. D 67:235-242 (2011)) was employed. The crystal structure of EphA4-LBD (PDBID:3CKH) and the crystal structure of the Fab region of IgG (PDBID:2VXT (L chain) and 1FGN (H chain)) were employed as search models for the molecular substitution method. The molecular model was constructed with the program COOT (Emsley P. et al., Acta Cryst. D 60: 2126-2132n (2004)) so that it fits the electron density obtained from the determined phase, and structure refinement was carried out with the program REFMAC (Murshudov G.N., Acta Cryst. D 53:240-255 (1997)).

A complex crystal structure of 2.0 Å resolution was obtained by the above structural calculation (R = 0.212, Rfree = 0.258).

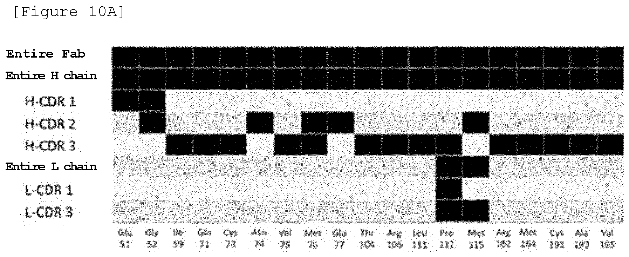

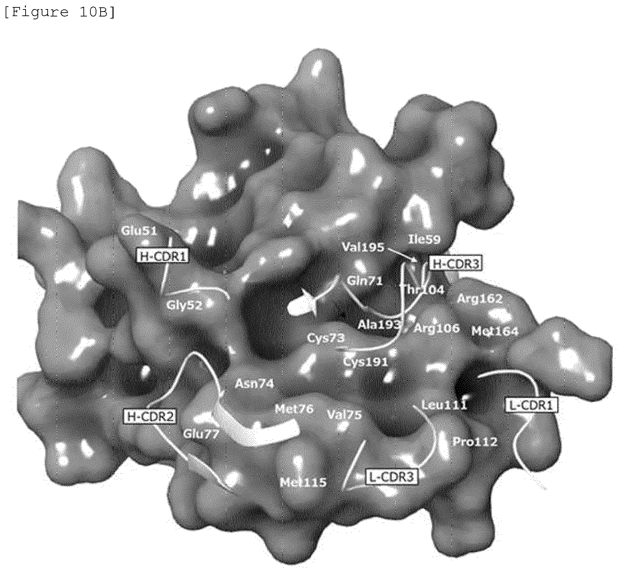

The crystal structure of the antibody A-Fab/EphA4-LBD complex obtained was analyzed with the interaction detection tool loaded in the computational chemistry system MOE 2018.0101 (Chemical Computing Group Inc.), and amino acid residues on EphA4-LBD that are in direct contact with antibody A-Fab were identified (Figure 10A) . The identified amino acid residues are Glu51, Gly52, Ile59, Gln71, Cys73, Asn74, Val75, Met76, Glu77, Thr104, Arg106, Leu111, Pro112, Met115, Arg162, Met164, Cys191, Ala193, and Val195. Figure 10B shows the surface structure of EphA4-LBD created with Maestro (version 11.0, Schrodinger, LLC). As a result, the present inventors concluded that the region where these amino acid residues exist is the antibody A-Fab binding region in EphA4-LBD.

### Example 1: Generation of humanized antibody of antibody A Preparation of humanized anti-EphA4 antibody

The variable region of the humanized antibody was designed. Based on the high homology against the framework region (FR) of antibody A, IGHV3-33*03 (SEQ ID NO. 42) and JH6 (SEQ ID NO. 43) for the heavy chain and IGKV1-17*01 (SEQ ID NO. 40) and JK4 (SEQ ID NO. 41) for the light chain were selected from among the FR of the human antibody as the FR of the humanized antibody. Then, employing the 3D structure prediction model of mouse antibody A, the amino acids in the FR that interact with the amino acids of the CDR were predicted, transplanted together with the CDRs of antibody A having Y32F mutation in CDR1 of the heavy chain (SEQ ID NO. 44, 27, 28, and 29-31), and HK2-42 (SEQ ID NO. 45) was designed as the heavy chain variable region of the humanized antibody and L1-8 (SEQ ID NO. 46) was designed as the light chain variable region of the humanized antibody. The transplanted the amino acid sequence of the CDR is shown in Table 2, and the nucleic acid sequence is shown in Table 3.

As the heavy chain constant region, the constant region of human IgG₂ (SEQ ID NO. 47) was employed. As the light chain constant region, human Igκ (SEQ ID NO. 48) was employed. Employing the Expi293 expression system (Gibco/Thermo Fisher), an expression vector comprising the gene sequence encoding the amino acid sequence of the humanized antibody (pcDNA 3.4) was transfected into Expi293F cells (Gibco/Thermo Fisher). As the gene sequence encoding the amino acid sequence of the humanized antibody, the nucleic acid sequence shown in SEQ ID NO. 55 was employed for the heavy chain variable region, the nucleic acid sequence shown in SEQ ID NO. 56 was employed for the light chain variable region, the nucleic acid sequence shown in SEQ ID NO. 57 was employed for the heavy chain constant region, and the nucleic acid sequence shown in SEQ ID NO. 58 was employed for the light chain constant region, respectively. The amino acid sequence of the heavy chain full length (not comprising the signal sequence) of the humanized antibody is the amino acid sequence shown in SEQ ID NO. 59, and the amino acid sequence of the light chain full length (not comprising the signal sequence) is the amino acid sequence shown in SEQ ID NO. 60. The nucleic acid sequence encoding the heavy chain full length of the humanized antibody is the nucleic acid sequence shown in SEQ ID NO. 61, and the nucleic acid sequence encoding the light chain full length is the nucleic acid sequence shown in SEQ ID NO. 62. The supernatant was collected, and MabSelect SuRe (GE Healthcare) was employed to purify the humanized antibody of antibody A (antibody B).

**[Table 2]**

| Amino acid sequence of CDRs of antibody B | |
|---|---|
| Name | Sequence |
| Heavy chain CDR1 | RFGVH (SEQ ID NO. 44) |
| Heavy chain CDR2 | VIWRGGSTDYNAAFMS (SEQ ID NO. 27) |
| Heavy chain CDR3 | ESLFGVYYDYGYYSMDY (SEQ ID NO. 28) |
| Light chain CDR1 | RASQEISGYLS (SEQ ID NO. 29) |
| Light chain CDR2 | AASTLDS (SEQ ID NO. 30) |
| Light chain CDR3 | LQYASYPLT (SEQ ID NO. 31) |

**[Table 3]**

| Nucleic acid sequence of CDRs of antibody B | |
|---|---|
| Name | Sequence |
| Heavy chain CDR1 | AGATTTGGAGTGCAT (SEQ ID NO. 49) |
| Heavy chain CDR2 | GTGATCTGGAGGGGAGGATCCACCGACTACAACGCTGCTTTTATGAGC (SEQ ID NO. 50) |
| Heavy chain CDR3 | GAGAGCCTGTTCGGCGTGTACTATGACTACGGCTACTATTCTATGGATTAT (SEQ ID NO. 51) |
| Light chain CDR1 | CGCGCCTCCCAGGAGATCTCTGGCTACCTGTCC (SEQ ID NO. 52) |
| Light chain CDR2 | GCTGCCTCCACCCTGGACTCT (SEQ ID NO. 53) |
| Light chain CDR3 | CTGCAGTACGCTTCCTATCCACTGACC (SEQ ID NO. 54) |

### Example 2: Affinity of humanized anti-EphA4 monoclonal antibody against human EphA4

The binding affinity of antibody B obtained in Example 1 against human EphA4 was determined by surface plasmon resonance (SPR method) employing Biacore T200 (GE Healthcare). First, anti-His antibodies (GE Healthcare, 28-9950-56) were fixed to a sensor chip CM5. Fixation was carried out by amine coupling method employing N-hydroxysuccinimide (NHS) and N-ethyl-N'-(3-dimethylaminopropyl) carbodiimide hydrochloride salt (EDC), and ethanolamine was employed for blocking (the sensor chip and reagents for fixation are all from GE Healthcare) . This was diluted with the fixation buffer (10 mM sodium acetate, pH 4.5) to 3.5 µg/mL, and fixed onto the sensor chip according to the protocol accompanying Biacore T200. Human EphA4 extracellular region-SEAP-His 10 was diluted with the running buffer HBS-EP (GE Healthcare, BR-1001-88), and the solution was sent and captured onto the flow cell for 120 seconds (about 10 RU of captured amount). Subsequently, antibody B serially diluted to a range of 100, 50, 25, 12.5, 6.3, 3.2, 1.6, 0 nM with HBS-EP was added to the sensor chip for 120 seconds, and the binding reaction curve at the time of addition (binding phase, 120 seconds) and after completing the addition (dissociation phase, 600 seconds) was sequentially observed. Upon completion of each observation, 4 M MgCl₂ (60 seconds, Wako Pure Chemicals) was added to regenerate the sensor chip. Fitting analysis by 1:1 binding model employing the software accompanying the system, BIA evaluation software, was carried out against the binding reaction curve obtained, and the affinity (KD = kd/ka) against human EphA4 was calculated.

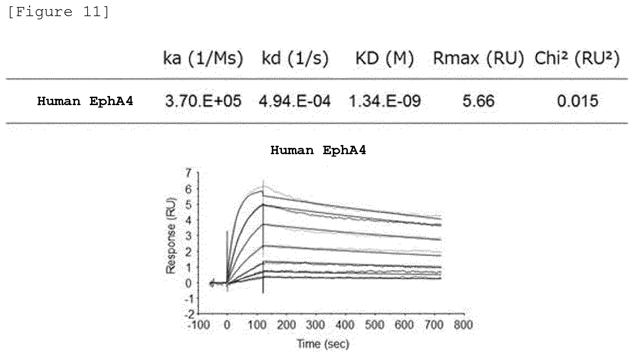

The binding affinity of antibody B against human EphA4 (KD value) was 1.34 × 10⁻⁹ M (Figure 11). It was found that antibody B shows affinity that is almost equivalent to that of antibody A before humanization.

### Example 3: EphA4 cleavage enhancing activity of humanized anti-EphA4 monoclonal antibody in hippocampus neurons

For antibody B obtained in Example 1, the evaluation of EphA4 cleavage enhancing activity employing hippocampus neurons was carried out according to the following steps.

Rat hippocampus neurons seeded in a 96-well dish (Falcon) were treated with antibody B (2.0, 6.7, 20 nM) and γ-secretase inhibitory drug Compound E (50 nM, Enzo Life Sciences), 24 hours thereafter washed with PBS (Wako Pure Chemicals), SDS sample buffer (Laemmli sample buffer (Bio-Rad) and 5% 2-mercaptoethanol (Bio-Rad)) was added and the cells were collected, and this was boiled for 5 minutes. SDS-PAGE was carried out with this sample, western blotting employing anti-EphA4 monoclonal antibodies (Abnova) was carried out, the band strength was quantified, and the value of EphA4 C-terminal fragment/full length EphA4 was calculated.

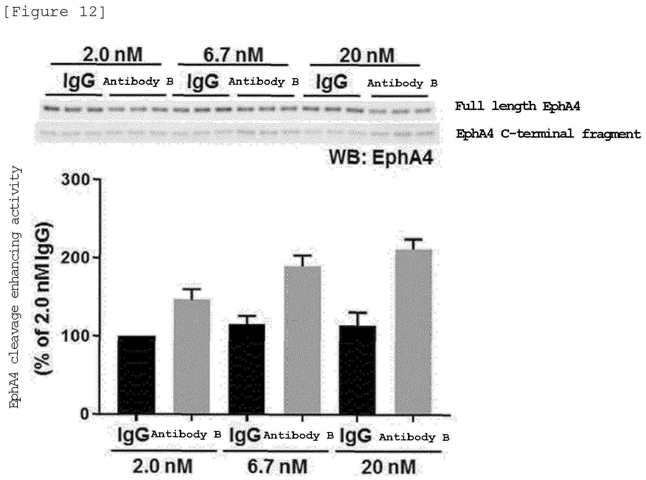

### Antibody B concentration-dependently enhanced EphA4 cleavage reaction in hippocampus neurons (Figure 12)

### Example 4: Human EphA4-human ligand binding inhibitory activity of humanized anti-EphA4 monoclonal antibody

For antibody B obtained in Example 1, the evaluation of binding inhibitory activity between human EphA4 and human ligand was carried out according to the following steps. Anti-alkaline phosphatase antibodies (Thermo SCIENTIFIC) were coated onto the wells of a 96-well plate (Nunc). After incubating at 4°C overnight, the wells were blocked at room temperature for 1 hour by 1% Block Ace (DS Pharma Biomedical). After washing 3 times with 0.05% Tween 20/PBS (Thermo SCIENTIFIC), human EphA4 extracellular region-SEAP-His protein (final concentration 10 nM) was seeded into the wells, and incubated at room temperature for 1 hour. After washing 3 times, the ligand and serially diluted antibody B (0, 0.003, 0.01, 0.03, 0.1, 0.3, 1, 3, 10, 30, 100, 300, 1000, 3000 nM) were supplemented to the wells. Note that biotinylated human EphrinA5-Fc chimera (R&D Systems, final concentration 0.7 nM) and biotinylated human EphrinB3-Fc chimera (R&D Systems, final concentration 2.3 nM) were employed as ligands. After incubating at room temperature for 1 hour and washing 3 times, horseradish peroxidase-labeled streptavidin (GE Healthcare) was added, and incubated at room temperature for 1 hour. After washing 3 times, TMBZ (3,3',5,5'-tetramethylbenzidine, Sigma) solution was added to the wells, and incubated at room temperature for 2-5 minutes. An equal amount of quenching solution (1 N H₂SO₄, Wako Pure Chemicals) was added to the wells, and absorbance at 450 nm was read by a microplate reader (Molecular Devices or PerkinElmer).

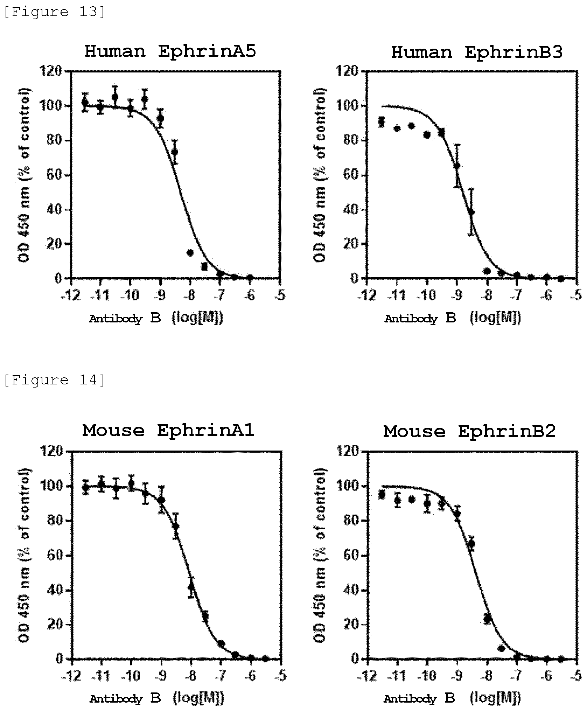

Antibody B concentration-dependently suppressed the binding between human EphA4 and human ligand, and the IC₅₀ values against human EphrinA5 and EphrinB3 binding were about 4.9 nM and 1.6 nM, respectively. Accordingly, it was found that antibody B strongly inhibits the binding between human EphA4 and human ligand, and shows inhibitory activity almost equivalent to that of antibody A before humanization (Figure 13).

### Example 5: Mouse EphA4-mouse ligand binding inhibitory activity of humanized anti-EphA4 monoclonal antibody

For antibody B obtained in Example 1, the evaluation of binding inhibitory activity between mouse EphA4 and mouse ligand was carried out according to the following steps. Anti-alkaline phosphatase antibodies (Thermo SCIENTIFIC) were coated onto the wells of a 96-well plate (Nunc). After incubating at 4°C overnight, the wells were blocked at room temperature for 1 hour by 1% Block Ace (DS Pharma Biomedical). After washing 3 times with 0.02% Tween 20/PBS (Thermo SCIENTIFIC), mouse EphA4 extracellular region-SEAP-His protein was added to the wells (final concentration 10 nM), and incubated at room temperature for 1 hour. After washing 3 times, the ligand and serially diluted antibody B (0, 0.003, 0.01, 0.03, 0.1, 0.3, 1, 3, 10, 30, 100, 300, 1000, 3000 nM) were supplemented to the wells. Note that biotinylated mouse EphrinA1-Fc chimera (R&D Systems, final concentration 6 nM) and biotinylated mouse EphrinB2-Fc chimera (R&D Systems, final concentration 2.5 nM) were employed as ligands. After incubating at room temperature for 1 hour and washing 3 times, horseradish peroxidase-labeled streptavidin (GE Healthcare) was added, and incubated at room temperature for 1 hour. After washing 3 times, TMBZ (3,3',5,5'-tetramethylbenzidine, Sigma) solution was added to the wells, and incubated at room temperature for 2 minutes. An equal amount of quenching solution (1 N H₂SO₄, Wako Pure Chemicals) was added to the wells, and absorbance at 450 nm was read by a microplate reader (Molecular Devices or PerkinElmer).

Antibody B concentration-dependently suppressed the binding between mouse EphA4 and mouse ligand, and the IC₅₀ values against mouse EphrinA1 and EphrinB2 binding were about 8.7 nM and 4.2 nM, respectively. Accordingly, it was found that antibody B strongly inhibits the binding between mouse EphA4 and mouse ligand, and shows inhibitory activity almost equivalent to that of antibody A before humanization (Figure 14).

### Example 6: Selectivity of humanized anti-EphA4 monoclonal antibody against human Eph receptor

Similarly to the method for preparing the mouse EphA4 extracellular region-SEAP-His protein described in Reference Example 1, DNA sequences encoding the signal sequence and the extracellular region of each Eph receptor (EphA1, EphA2, EphA3, EphA4, EphA5, EphA6, EphA7, EphA8, EphA10, EphB1, EphB2, EphB3, EphB4, EphB6) of human were amplified by RT-PCR employing total RNA derived from tissue, and cloned into a pENTRlA vector (Invitrogen/Life Technologies) having a DNA sequence encoding SEAP protein and a histidine tag. Next, a DNA sequence encoding the signal sequence and the extracellular region of each Eph receptor of human, SEAP protein, and a histidine tag was transferred to a pcDNA 3.1 rfcB vector by the LR reaction of the Gateway System (Invitrogen/Life Technologies), and a vector expressing a protein having SEAP protein and a His tag fused to the extracellular region of each Eph receptor of human (referred to as "Eph receptor extracellular region-SEAP-His protein") (referred to as "Eph receptor extracellular region-SEAP-His protein expression vector") was constructed.

Next, employing the Expi293 expression system (Gibco/Thermo Fisher), expression vectors for each Eph receptor of human extracellular region-SEAP-His protein were introduced into Expi293F cells (Gibco/Thermo Fisher). After 5 days of incubation (5% CO₂, 37°C), the culture supernatant was collected, and centrifuged at room temperature at 1500 rpm for 5 minutes. The centrifugation supernatant was filtered with a 0.45 µm filter (Millipore).

For antibody B obtained in Example 1, the evaluation of the binding activity of human Eph receptor was carried out according to the following steps.

Rabbit anti-6-His antibodies (Bethyl Laboratories) were coated onto the wells of a 96-well plate (Nunc). After incubating at 4°C overnight, the wells were blocked at room temperature for 1 hour by 1% Block Ace (DS Pharma Biomedical) . After washing 3 times with 0.05% Tween 20/PBS (Thermo SCIENTIFIC), each Eph receptor of human extracellular region-SEAP-His protein (final concentration 1 nM) was seeded into each well, and incubated at room temperature for 1 hour. After washing 3 times, human IgG solution (100 µg/mL, Mitsubishi Pharma Corporation) and antibody B (10 µg/mL) were supplemented to the wells, and incubated at room temperature for 1 hour. Horseradish peroxidase-labeled donkey anti-human IgG antibody (Jackson ImmunoResearch Laboratories) was added, and incubated at room temperature for 1 hour. After washing 3 times, TMBZ (3,3',5,5'-tetramethylbenzidine, Sigma) solution was added to the wells, and upon confirmation of modest coloring, an equal amount of quenching solution (1 N H₂SO₄, Wako Pure Chemicals) was added to the wells, and absorbance at 450 nm was read by a microplate reader (PerkinElmer) .

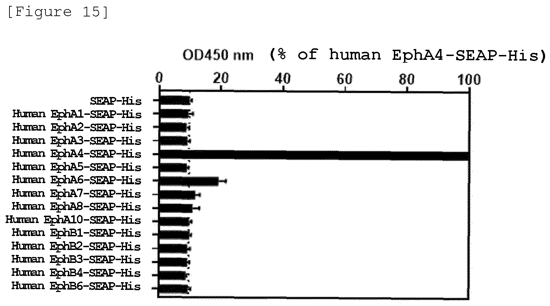

It was fount that similarly to antibody A before humanization, antibody B specifically binds to human EphA4 among the human Eph receptor family (Figure 15).

### Example 7: Selectivity of humanized anti-EphA4 monoclonal antibody against mouse Eph receptor

According to the method for preparing the EphA4 extracellular region-Fc-His protein described in Reference Example 1, DNA sequences encoding the signal sequence and the extracellular region of each Eph receptor (EphA1, EphA3, EphA4, EphA5, EphA6, EphA7, EphA8, EphA10, EphB1, EphB2, EphB3, EphB4, EphB6) of mouse were amplified by RT-PCR employing total RNA derived from tissue, and cloned into a pENTRlA vector (Invitrogen/Life Technologies) having a DNA sequence encoding the Fc region of human IgG₁ and a histidine tag. Next, a DNA sequence encoding the signal sequence and the extracellular region of each Eph receptor (EphA1, EphA3, EphA4, EphA5, EphA6, EphA7, EphA8, EphA10, EphB1, EphB2, EphB3, EphB4, EphB6) of mouse, Fc, and a histidine tag was transferred to a pcDNA 3.1_rfcB vector by the LR reaction of the Gateway System (Invitrogen/Life Technologies), and expression vectors for each Eph receptor of mouse extracellular region-Fc-His protein were constructed. For construction of mouse EphA2 extracellular region-Fc-His protein expression vector, a DNA sequence encoding the signal sequence and the extracellular region of mouse EphA2 was amplified by RT-PCR employing total RNA derived from tissue, cloned into a pcDNA 3.1 vector having a DNA sequence encoding Fc and a histidine tag, and mouse EphA2 extracellular region-Fc-His protein expression vector was constructed.

Next, employing the Expi293 expression system (Gibco/Thermo Fisher), expression vectors for each Eph receptor of mouse extracellular region-Fc-His protein were introduced into Expi293F cells (Gibco/Thermo Fisher) . After 5 days of culture (5% CO2, 37°C, 120 rpm), the culture supernatant was collected, and centrifuged at room temperature at 1500 rpm for 5 minutes. The centrifugation supernatant was filtered with a 0.45 µm filter (Millipore).

For antibody B, the evaluation of the binding activity of mouse Eph receptor was carried out according to the following steps.

Rabbit anti-6-His antibodies (Bethyl Laboratories) were coated onto the wells of a 96-well plate (Nunc). After incubating at 4°C overnight, the wells were blocked at room temperature for 1 hour by 1% Block Ace (DS Pharma Biomedical) . After washing 3 times with 0.05% Tween 20/PBS (Thermo SCIENTIFIC), each Eph receptor of mouse extracellular region-Fc-His protein (final concentration 1 nM) was seeded into each well, and incubated at room temperature for 1 hour. After washing 3 times, human IgG solution (100 µg/mL, Sigma) and antibody B (10 µg/mL) were supplemented to the wells, and incubated at room temperature for 1 hour. Horseradish peroxidase-labeled goat anti-human Kappa Light Chain antibody (IBL) was added, and incubated at room temperature for 1 hour. After washing 3 times, TMBZ (3,3',5,5'-tetramethylbenzidine, Sigma) solution was added to the wells, and upon confirmation of modest coloring, an equal amount of quenching solution (1 N H₂SO₄, Wako Pure Chemicals) was added to the wells, and absorbance at 450 nm was read by a microplate reader (PerkinElmer).

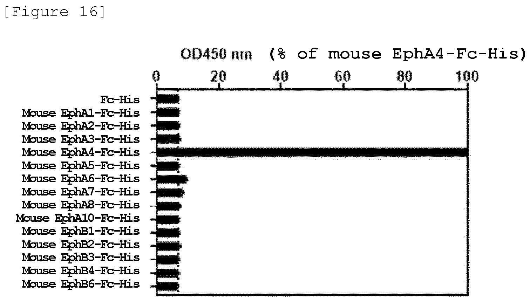

Antibody B had specific binding activity only to mouse EphA4 among the mouse Eph receptor family (Figure 16).

### Example 8: Reactivity of humanized anti-EphA4 monoclonal antibody against mouse, rat, monkey, and human EphA4

For antibody B, the evaluation of binding activity with various EphA4 was carried out according to the following steps.

Anti-alkaline phosphatase antibodies (Thermo SCIENTIFIC) were coated onto the wells of a 96-well plate (Nunc). After incubating at 4°C overnight, the wells were blocked at room temperature for 1 hour by 1% Block Ace (DS Pharma Biomedical) . After washing 3 times with 0.05% Tween 20/PBS (Thermo SCIENTIFIC), mouse, rat, monkey, and human EphA4 extracellular region-SEAP-His proteins (final concentration 1 nM) were seeded into the wells, and incubated at room temperature for 1 hour. After washing 3 times, human IgG solution (100 µg/mL, Mitsubishi Pharma Corporation) and antibody B (0, 0.00013, 0.00064, 0.0032, 0.016, 0.08, 0.4, 2, 10 µg/mL) were supplemented to the wells, and incubated at room temperature for 1 hour. Horseradish peroxidase-labeled donkey anti-human IgG antibody (Jackson ImmunoResearch Laboratories) was added, and incubated at room temperature for 1 hour. After washing 3 times, TMBZ (3,3',5,5'-tetramethylbenzidine, Sigma) solution was added to the wells, and upon confirmation of modest coloring, an equal amount of quenching solution (1 N H₂SO₄, Wako Pure Chemicals) was added to the wells, and absorbance at 450 nm was read by a microplate reader (PerkinElmer) .

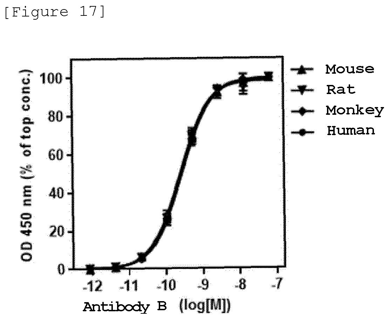

Antibody B had equivalent binding activity in any of mouse, rat, monkey, and human EphA4 (Figure 17).

### Example 9: Reactivity of humanized anti-EphA4 monoclonal antibody against human EphA4 extracellular region, ligand binding domain, fibronectin type III domain 1, and fibronectin type III domain 2

For antibody B obtained in Example 1, the evaluation of binding activity with various domains in human EphA4 was carried out according to the following steps.

Rabbit anti-6-His antibodies (Bethyl Laboratories) were coated onto the wells of a 96-well plate (Nunc). After incubating at 4°C overnight, the wells were blocked at room temperature for 1 hour by 1% Block Ace (DS Pharma Biomedical). After washing twice with 0.02% Tween 20/PBS (Nacalai Tesque), human EphA4 extracellular region-MBP-His protein, human EphA4 ligand binding domain-MBP-His protein, human EphA4 fibronectin type III domain 1-MBP-His protein, and human EphA4 fibronectin type III domain 2-MBP-His protein (final concentration 10 nM) were seeded into the wells, and incubated at room temperature for 1 hour. After washing 3 times, antibody B (final concentration 10 nM) was supplemented to the wells, and incubated at room temperature for 1 hour. Horseradish peroxidase-labeled rabbit anti-human IgG Fcy fragment antibody (Jackson ImmunoResearch Laboratories) was added, and incubated at room temperature for 1 hour. After washing 5 times, TMB (KPL) solution was added to the wells, and upon confirmation of modest coloring, an equal amount of quenching solution (2 N H₂SO₄, Wako Pure Chemicals) was added to the wells, and absorbance at 450 nm and 650 nm were read by a microplate reader (PerkinElmer).

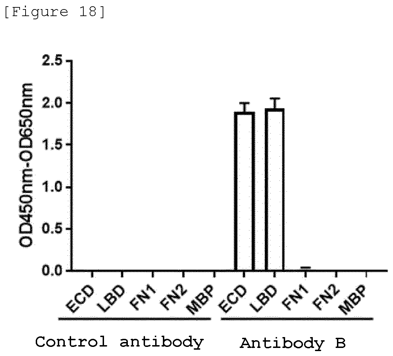

Antibody B had binding activity with human EphA4 extracellular region (ECD) and ligand binding domain (LBD) (Figure 18). It did not react to fibronectin type III domain 1 (FN1) and fibronectin type III domain 2 (FN2) . Accordingly, it was found that antibody B specifically binds to the ligand binding domain of human EphA4 extracellular region.

### Example 10: Increasing effect of humanized anti-EphA4 monoclonal antibody against number of spines in hippocampus neuron

Preparation of rat hippocampus neurons was carried out as described in Reference Example 1 (B). The EGFP gene was introduced into rat hippocampus neurons with Nucleofector (Lonza), and seeded in a 24-well plate (Falcon) containing a cover glass (Matsunami Glass Ind.,Ltd.) coated with poly L-lysine.

The counting of spine employing hippocampus neurons was carried out according to the following steps. EGFP-introduced rat hippocampus neurons at Day 13 of culture that were seeded in a 24-well plate (Falcon) containing a cover glass (Matsunami Glass Ind.,Ltd.) coated with poly L-lysine were treated with control antibody (human; Sigma) or antibody B (6.7, 20 nM) for 24 hours. Then, the cover glass was transferred to 2% PFA (Wako Pure Chemicals) /4% Sucrose (Wako Pure Chemicals) /PBS, and left still for 20 minutes to fix the cells. After removing the fixing solution and washing the cells 3 times with PBS, 0.25% TritonX-100 (Wako Pure Chemicals)/PBS was added, and cell permeabilization was carried out for 15 minutes. After removing the solution, transferring the cover glass to 2% BSA (Sigma)/0.25% TritonX-100/OPTI-MEM (GIBCO), and subjecting to 1 hour of blocking, anti-GFP antibody (Nacalai Tesque) was reacted for 1 hour 30 minutes. After removing the primary antibody solution and washing 3 times with PBS, the secondary antibody was reacted for 1 hour. After removing the secondary antibody solution and washing 3 times with PBS, Prolong Gold antifade reagent (Molecular probes) was added and sealed, and observation was carried out with LSM800 (ZEISS). The experiment described above was carried out 3 times, neurons were extracted from two cover glasses per experiment, the spines present on each dendrite were counted with an image analysis software Imaris^{™} (Bitplane), and the number of spines per 10 µm in each neuron was calculated.

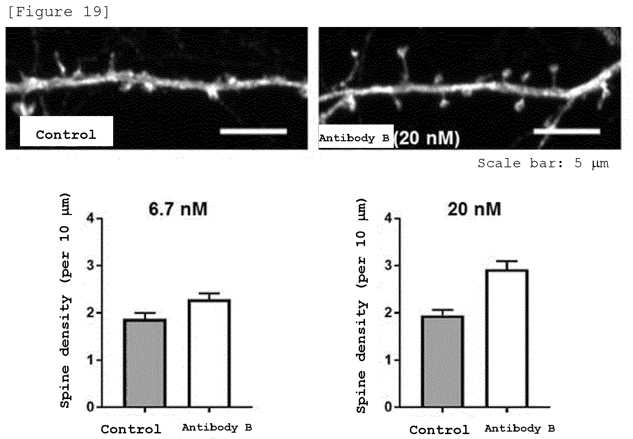

Antibody B increased the number of spines in the hippocampus neuron (Figure 19). This result shows that antibody B has the activity to stabilize the spine in hippocampus neurons.

### Example 11: Human EphA4 cleavage enhancing activity of humanized anti-EphA4 monoclonal antibody

For antibody B obtained in Example 1, the evaluation of the cleavage enhancing activity against human EphA4 was carried out according to the following steps.

Preparation of rat hippocampus neurons was carried out as described in Reference Example 1 (B). A human EphA4-HA protein expression vector was introduced into rat hippocampus neurons with Nucleofector (Lonza), and seeded in a 96-well dish (Falcon) coated with poly L-lysine. The seeded rat hippocampus neurons were treated with antibody B (6.7, 20, 67 nM) and γ-secretase inhibitory drug Compound E (50 nM, Enzo Life Sciences), about 24 hours thereafter washed with PBS (Wako Pure Chemicals), SDS sample buffer (Laemmli sample buffer (Bio-Rad) and 5% 2-mercaptoethanol (Bio-Rad)) was added and the cells were collected, and this was boiled for 5 minutes. SDS-PAGE was carried out with this sample, western blotting employing rat anti-HA monoclonal antibody (Roche) was carried out, the band strength was quantified, and the value of EphA4 C-terminal fragment/full length EphA4 was calculated.

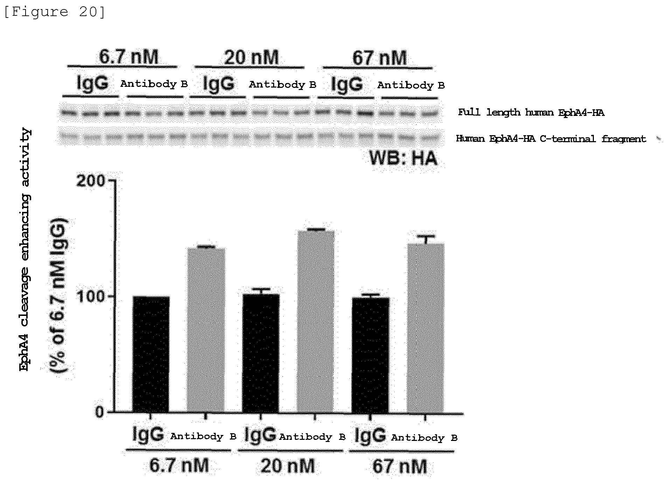

Antibody B enhanced human EphA4 cleavage reaction in hippocampus neurons (Figure 20).

### Example 12: Involvement of MMP and ADAM on increasing effect of humanized anti-EphA4 monoclonal antibody for number of spines in hippocampus neuron

Preparation of rat hippocampus neurons was carried out as described in Reference Example 1 (B). The EGFP gene was introduced into some rat hippocampus neurons with Nucleofector (Lonza), and seeded in a 24-well plate (Falcon) containing a cover glass (Matsunami Glass Ind.,Ltd.) coated with poly L-lysine.

The counting of spine employing hippocampus neurons was carried out according to the following steps. EGFP-introduced rat hippocampus neurons at Day 13 of culture that were seeded in a 24-well plate (Falcon) containing a cover glass (Matsunami Glass Ind.,Ltd.) coated with poly L-lysine were treated with control antibody (human IgG2; Sigma) or antibody B (20 nM) and DMSO (Sigma) or GM6001 which is an inhibitor of MMP and ADAM (2.5 µM, MedChemExpress) for 24 hours. Then, the cover glass was transferred to 2% PFA (Wako Pure Chemicals) /4% Sucrose (Wako Pure Chemicals) /PBS, and left still for 20 minutes to fix the cells. After removing the fixing solution and washing the cells 3 times with PBS, 0.25% TritonX-100 (Wako Pure Chemicals)/PBS was added, and cell permeabilization was carried out for 15 minutes. After removing 0.25% TritonX-100/PBS, transferring the cover glass to 2% BSA (Sigma)/0.25% TritonX-100/OPTI-MEM (GIBCO), and subjecting to 1 hour of blocking, anti-GFP antibody (Nacalai Tesque) was reacted for 1 hour 30 minutes. After removing the primary antibody solution and washing 3 times with PBS, the secondary antibody was reacted for 1 hour. After removing the secondary antibody solution and washing 3 times with PBS, Prolong Gold antifade reagent (Molecular probes) was added and sealed, and observation was carried out with LSM800 (ZEISS). The experiment described above was carried out 3 times, neurons were extracted from two cover glasses per experiment, the spines present on each dendrite were counted with an image analysis software Imaris^{™} (Bitplane), and the number of spines per 10 µm in each neuron was calculated.

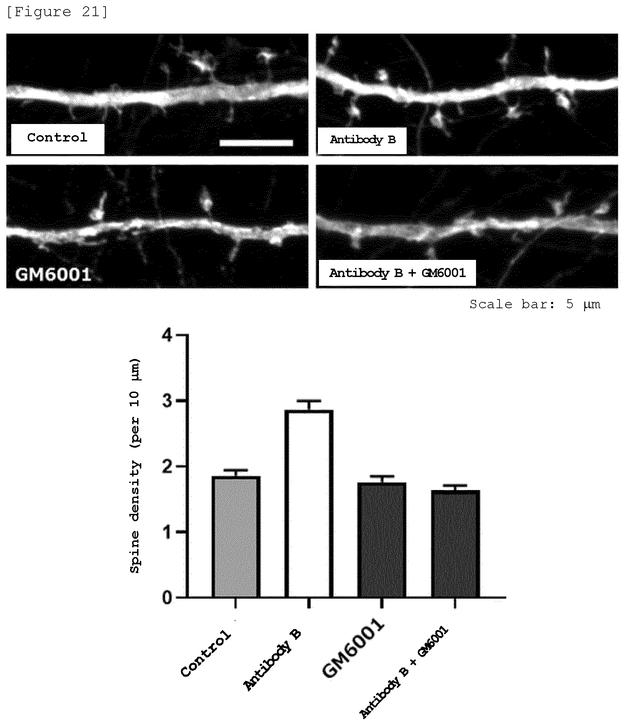

The increase in the number of spines in the hippocampus neuron by antibody B was inhibited by simultaneous treatment with GM6001 (Figure 21). This result shows that antibody B has spine stabilization activity via MMP and ADAM in hippocampus neurons.

### Example 13: Human iPS cell-derived motor neuron protection effect of humanized anti-EphA4 monoclonal antibody in in vitro ALS model

### (A) Maintenance culture of human iPS cells

Maintenance culture of human iPS cells was carried out according to the following steps. Human iPS cells (201B7) cryopreserved in liquid nitrogen at Stem cell banker (Takara) were removed from the liquid nitrogen gas layer, and suspended and dissolved in 5 mL of human iPS cell culture medium (Essential 8, Thermo Fisher Scientific) warmed to 37°C in advance. The cell suspension was collected in a 15 mL conical tube (Thermo Fisher Scientific), and after centrifugation at 1000 rpm for 5 minutes at room temperature, the supernatant was removed, suspended in fresh medium, and then seeded in a ϕ60 mm cell culture dish (Corning) coated with 0.3 µg/cm² iMatrix-511 (Nippi) in advance, 10 µM Y-27632 (WAKO) was added, after which it was cultured in a CO₂ incubator (37°C, 5% CO₂). The medium was exchanged every day, and passage culture was carried out upon reaching subconfluency to carry out the maintenance culture of human iPS cells. The passage culture was carried out as follows. The culture medium of human iPS cells in a subconfluent state was aspirated, washed with 2 mL of PBS (WAKO), and then Accutase (Nacalai Tesque) 1 mL was added and incubated in a CO₂ incubator (37°C, 5% CO₂) for 5 minutes. Human iPS cells were dissociated into single cells by suspending in 4 mL of human iPS cell culture medium comprising 10 µM Y-27632, then collected in a 15 mL conical tube. Centrifugation at 1000 rpm for 5 minutes at room temperature was carried out, the supernatant was aspirated, and then the human iPS cells were suspended in 1 mL of human iPS cell culture medium comprising 10 µM Y-27632. The number of cells was counted, 2 x 10⁵ human iPS cells were suspended in 4 mL of human iPS cell culture medium, and then seeded in a ϕ60 mm cell culture dish coated with 0.3 µg/cm² iMatrix-511, and cultured in a CO₂ incubator (37°C, 5% CO₂). Human iPS cells that have undergone passage culture once or more times were employed for the experiment.

### (B) Establishment and maintenance culture of astrocytes

Establishment and maintenance culture of astrocytes from neonatal mouse was carried out according to the following steps. A wild-type neonatal mouse (C57BL/6JJmsSlc (Japan SLC, Inc.)) at two days after birth and a neonatal crossbreed mouse between wild-type mouse and mutated human SOD1 (G93A) Tg-(B6.Cg-Tg (SOD1 G93A) 1Gur/J (Jackson Laboratories)) mouse were euthanized by decapitation under isoflurane (Intervet K.K.), and then the cerebral cortex was isolated and dispersed by treatment with 0.25% trypsin-EDTA (Thermo Fisher Scientific) at 37°C for 15 minutes. After enzyme treatment, this was diluted with 4 mL of Dulbecco's Modified Eagle Medium (Thermo Fisher Scientific) (10% FBS-DMEM) containing 10% FBS (Thermo Fisher Scientific) and 1% penicillin streptomycin (Nacalai Tesque) to stop enzyme digestion. Then, impurities other than single cells were filtered by a cell strainer (Corning), and centrifuged at 1500 rpm for 5 minutes. The supernatant was aspirated, the cells were diluted in 4 mL of fresh 10% FBS-DMEM, seeded in a ϕ60 mm cell culture dish for each individual, and cultured at 37°C. The medium was aspirated two days after seeding, and 4 mL of fresh 10% FBS-DMEM was added to the cells to carry out medium exchange. Upon reaching confluency, passage culture was carried out. Passage culture of astrocytes derived from neonatal mouse was carried out as follows. The 10% FBS-DMEM of the astrocytes after reaching confluency on the ϕ60 mm cell culture dish was aspirated, and then washed with 2 mL of PBS (WAKO), 1 mL of 0.25% trypsin-EDTA was added and incubated for 3 minutes in a CO₂ incubator (37°C, 5% COz) . The astrocytes were dissociated into single cells by suspending with 3 mL of 10% FBS-DMEM, and then collected in a 15 mL conical tube. This was centrifuged at 1500 rpm for 3 minutes at room temperature, the supernatant was aspirated, and then 8 mL of fresh 10% FBS-DMEM was added to the cells, and seeded in a cp100 mm cell culture dish (passage 1). Passage culture was carried out with a method similar to the when the cells reached confluency. Note that when carrying out passage culture of astrocytes cultured in a cp100 mm cell culture dish, 4 mL of PBS and 2 mL of 0.25% trypsin-EDTA were used. Moreover, when carrying out passage culture, a part of the cell suspension was collected and subjected to genotyping of mutated human SOD1 (G93A) . The astrocytes after carrying out passage culture for a total of 3 times were diluted with Cell banker (NIPPON ZENYAKU KOGYO CO.,LTD.) and cryopreserved at -80°C until testing. Upon testing, the cryopreserved cell suspensions were each dissolved in a thermostat bath, and then diluted with 10% FBS-DMEM warmed to 37°C in advance. After centrifugation of each cell suspension (1500 rpm, 3 minutes, room temperature), the supernatant was removed, suspended in fresh medium, and then seeded in an 8-well chamber (ibidi), and maintenance culture was carried out in a CO₂ incubator (37°C, 5% CO₂).

The genotyping of mutated human SOD1 (G93A) was carried out employing REDExtract-N-Amp^{™} Tissue PCR kit (Sigma). The cell suspension collected when carrying out passage culture of astrocytes was transferred to a 1.5 mL tube, and centrifuged at 1500 rpm for 3 minutes. After centrifugation, the supernatant was aspirated, 1 mL of PBS was added to the cells and washed, centrifuged again, and then aspirated. Fifty microliters of the extracted solution and 12.5 µL of the tissue preparation solution were mixed and added to the sample. After mixing, this was transferred to a polymerase chain reaction (PCR) tube, reacted in GeneAmp^{™} PCR system 9700 (Applied biosystems^{™}) at 55°C for 10 minutes and at 95°C for 3 minutes, and then 50 µL of the neutralization solution accompanying the kit was added to prepare genome DNA.

The extracted genome DNA was employed to carry out genomic PCR with the composition shown in Table 4. The primer sequences used in the PCR are shown in Table 5. After PCR, electrophoresis was carried out with 1% agarose gel/100V/20 minutes. Those with two bands detected, the internal standard at 324 bp and the mutated human SOD1 (G93A) at 236 bp, were identified as mutated human SOD1 (G93A)-expressing astrocytes.

**[Table 4]**

| Genomic PCR Mixture | |
|---|---|
| Reagent name | Liquid amount |
| Template: | 1 µL |
| Red mix: | 5 µL |
| Primer 1 (100 µmol/L): | 0.05 µL |
| Primer 2 (100 µmol/L): | 0.05 µL |
| Primer 3 (100 µmol/L): | 0.05 µL |
| Primer 4 (100 µmol/L): | 0.05 µL |
| Distilled water: | 3.8 µL |
| Total: | 10 µL |

| | |
|---|---|
| Red mix = REDExtract-N-Amp PCR reaction mix. | |

**[Table 5]**

| Nucleotide sequence of primers | | |
|---|---|---|
| Primer 1 | Mutated human SOD1 (G93A) | CATCAGCCCTAATCCATCTGA (SEQ ID NO. 63) |
| Primer 2 | Mutated human SOD1 (G93A) | CGCGACTAACAATCAAAGTGA (SEQ ID NO. 64) |
| Primer 3 | Internal standard | CTAGGCCACAGAATTGAAAGATCT (SEQ ID NO. 65) |
| Primer 4 | Internal standard | GTAGGTGGAAATTCTAGCATCATCC (SEQ ID NO. 66) |

### (C) Evaluation of human iPS cell-derived motor neuron protection effect in in vitro ALS model

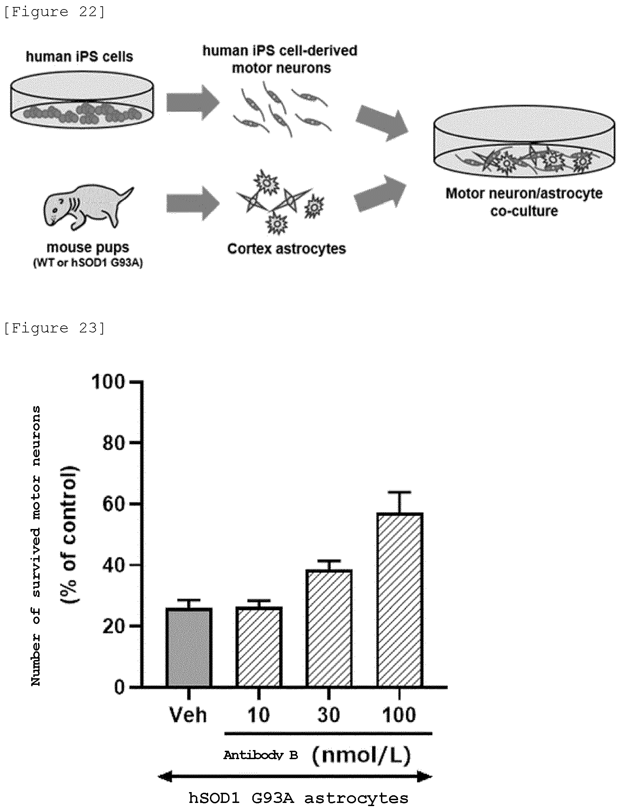

The evaluation of human iPS cell-derived motor neuron protection effect in *in vitro* ALS model was carried out according to the following steps. After obtaining single cells suspension of human iPS cells with a method similar to the passage culture in the above (A), centrifugation at 1000 rpm for 5 minutes at room temperature was carried out, and the supernatant was aspirated. After suspending the human iPS cells in a DFK20 medium (DMEM/F12 (Thermo Fisher Scientific) comprising 20% Knockout serum replacement (KSR, Thermo Fisher Scientific), 1% Non-essential amino acid (NEAA, Thermo Fisher Scientific), 1% GlutaMAX-I Supplement (Thermo Fisher Scientific), 100 units/mL penicillin - 100 µg/mL streptomycin (Nacalai Tesque), and 100 µM β-mercaptoethanol (Thermo Fisher Scientific)), the number of cells was counted. Three x 10⁵ human iPS cells was suspended in 2 mL of DFK20 medium comprising 10 µM SB431542 (Sigma), 100 nM LDN193189 (Sigma), 3 µM CHIR99021 (Cayman Chemical), 10 µM Y-27632, seeded in one well of a low adhesive 6-well cell culture plate (Corning), and cultured in a CO₂ incubator (37°C, 5% CO₂). On Day 3 of culture, human iPS cell differentiated cell aggregates (SFEBs) were collected in a 15 mL conical tube together with the medium, and centrifuged at 300 rpm for 2 minutes at an ordinary temperature in order to precipitate the cell mass. This supernatant was aspirated, SFEBs was gently suspended in a DFK20 medium comprising 10 µM SB431542, 100 nM LDN193189, 3 µM CHIR99021 (Cayman), 5 µM Y-27632, and 1 µM Retinoic Acid (Sigma), and returned to the original well to carry out medium exchange. Medium exchange was carried out also on Day 5 of culture with a similar method. Note that medium exchange was carried out with the concentration of Y-27632 at 2.5 µM (other compounds were the same as Day 3 of culture) . On Day 7 of culture, SFEBs was collected in a 15 mL conical tube together with the medium, and left still for 10 minutes at an ordinary temperature in order to precipitate SFEBs. This supernatant was aspirated, SFEBs was suspended in 3 mL of DFK5 medium (DMEM/F12 comprising 5% KSR, 1% NEAA, 1% GlutaMAX-I Supplement, 100 units/mL penicillin - 100 µg/mL streptomycin, and 100 µM β-mercaptoethanol) comprising 1 µM Retinoic Acid, 1 µM Purmorphamine (Myltenyi Biotech) and returned to the original well, and cultured in a CO₂ incubator (37°C, 5% COz) . Then, medium exchange was carried out with the same steps as in Day 7 of culture every 2-3 days, and human iPS cells were differentiation induced into motor neurons. On Day 33 of culture, SFEBs was collected in a 15 mL conical tube together with the medium, and left still for 5 minutes at an ordinary temperature in order to precipitate SFEBs. The supernatant was aspirated, 2 mL of Accutase comprising 10 µM Y-27632 was added, and incubated in a 37°C thermobath for 10 minutes. Then, the cell mass was dispersed by pipetting 30 times with a P1000 pipette, and then the enzyme reaction was stopped with 10 mL of DFK5 medium comprising 10 µM Y-27632. The cell suspension was collected in a fresh 15 mL conical tube, centrifuged at 1000 rpm for 5 minutes at room temperature, and the supernatant was aspirated. After resuspending the cells with a DFK5 medium comprising 10 µM Y-27632, this was filtered with a cell strainer (Corning), and then the number of cells was counted. The cells were prepared to a suspension of 5 x 10⁵ cells/mL of suspension with a co-culture medium (Neurobasal medium (Thermo Fisher Scientific) comprising 2% B27 Supplement (Thermo Fisher Scientific), 10 µM Y-27632, 1% GlutaMax-I Supplement, 100 units/mL penicillin - 100 µg/mL streptomycin), and divided into the control group, the vehicle addition group, and the drug treatment group. The co-culture medium was employed for diluting the drug. After adding the vehicle (co-culture medium) and the drug which is antibody B to each group, they were seeded at 200 µL/well in a 8-well chamber seeded with 8 x 10⁴ cells/well of mouse-derived wild-type astrocytes or mutated human SOD1 (G93A)-expressing astrocytes in advance, and used for evaluation as a co-cultured cells of astrocytes and motor neurons. The number of motor neurons observed with the co-culture of wild-type astrocytes and motor neurons was set as the control. In the vehicle addition group and the drug treatment group, co-culture of mutated human SOD1 (G93A) -expressing astrocytes and motor neurons was carried out, and the conditions were set as vehicle addition (1% co-culture medium) and antibody B (10, 30, 100 nmol/L). After culturing for 2 days under each condition in a CO₂ incubator (37°C, 5% CO₂), motor neuron cells were immunocytochemically stained with anti-ISL1 antibody (Abcam) and anti-Human Nuclear Antigen (HNA) antibody (Millipore). ISL1/HNA co-positive cells per well were counted as survived motor neurons, and the motor neuron survival rate was calculated as % against the control. Figure 22 shows a simple schematic diagram showing the evaluation system steps.

In mutated human SOD1 (G93A)-expressing astrocyte/human iPS cell-derived motor neuron co-culture (in *vitro* ALS model), the motor neuron survival rate was significantly reduced. Antibody B concentration-dependently suppressed human iPS cell-derived motor neuron death induced by mutated human SOD1 (G93A)-expressing astrocytes (Figure 23). This result shows that antibody B promotes the survival of motor neurons in *in vitro* ALS model.

## Claims

1. A pharmaceutical composition for treating amyotrophic lateral sclerosis (ALS) comprising an anti-EphA4 antibody, wherein
the anti-EphA4 antibody comprises a heavy chain comprising:
(a) a heavy chain CDR1 consisting of the amino acid sequence shown in SEQ ID NO. 44;
(b) a heavy chain CDR2 consisting of the amino acid sequence shown in SEQ ID NO. 27; and
(c) a heavy chain CDR3 consisting of the amino acid sequence shown in SEQ ID NO. 28; and
a light chain comprising:
(d) a light chain CDR1 consisting of the amino acid sequence shown in SEQ ID NO. 29;
(e) a light chain CDR2 consisting of the amino acid sequence shown in SEQ ID NO. 30; and
(f) a light chain CDR3 consisting of the amino acid sequence shown in SEQ ID NO. 31.

2. The pharmaceutical composition according to claim 1, wherein the anti-EphA4 antibody is humanized.

3. The pharmaceutical composition according to claim 1 or 2, wherein the anti-EphA4 antibody specifically binds to EphA4 and enhances the cleavage of EphA4.

4. The pharmaceutical composition according to any one of claims 1 to 3, wherein the anti-EphA4 antibody specifically binds to EphA4 and inhibits the binding between EphA4 and ephrin.

5. The pharmaceutical composition according to any one of claims 1 to 4, wherein
the heavy chain comprises a variable region consisting of the amino acid sequence shown in SEQ ID NO. 45, and
the light chain comprises a variable region consisting of the amino acid sequence shown in SEQ ID NO. 46.

6. The pharmaceutical composition according to any one of claims 1 to 5, wherein the constant region of the heavy chain and the constant region of the light chain comprise an amino acid sequence derived from a human antibody.

7. The pharmaceutical composition according to claim 6, wherein the constant region of the heavy chain is the constant region of human IgG.

8. The pharmaceutical composition according to claim 7, wherein the constant region of human IgG is the constant region of human IgG₂.

9. The pharmaceutical composition according to claim 8, wherein the constant region of human IgG₂ comprises the amino acid sequence shown in SEQ ID NO. 47.

10. The pharmaceutical composition according to any one of claims 6 to 9, wherein the constant region of the light chain is the constant region of human Igκ.

11. The pharmaceutical composition according to claim 10, wherein the constant region of human Igκ comprises the amino acid sequence shown in SEQ ID NO. 48.

12. A pharmaceutical composition for treating amyotrophic lateral sclerosis (ALS) comprising an anti-EphA4 antibody, wherein
the anti-EphA4 antibody comprises a heavy chain and a light chain,
the heavy chain comprises the amino acid sequence shown in SEQ ID NO. 59,
the light chain comprises the amino acid sequence shown in SEQ ID NO. 60, and
the C-terminal lysine of the heavy chain may be optionally deleted.

13. The pharmaceutical composition according to claim 12, wherein the C-terminal lysine of the heavy chain is deleted.

14. The use of an anti-EphA4 antibody for manufacturing a pharmaceutical composition for treating amyotrophic lateral sclerosis (ALS), wherein
the anti-EphA4 antibody comprises a heavy chain comprising:
(a) a heavy chain CDR1 consisting of the amino acid sequence shown in SEQ ID NO. 44;
(b) a heavy chain CDR2 consisting of the amino acid sequence shown in SEQ ID NO. 27; and
(c) a heavy chain CDR3 consisting of the amino acid sequence shown in SEQ ID NO. 28; and
a light chain comprising:
(d) a light chain CDR1 consisting of the amino acid sequence shown in SEQ ID NO. 29;
(e) a light chain CDR2 consisting of the amino acid sequence shown in SEQ ID NO. 30; and
(f) a light chain CDR3 consisting of the amino acid sequence shown in SEQ ID NO. 31.

15. The use of the anti-EphA4 antibody according to claim 14, wherein
the heavy chain comprises a variable region consisting of the amino acid sequence shown in SEQ ID NO. 45, and
the light chain comprises a variable region consisting of the amino acid sequence shown in SEQ ID NO. 46.
